(19) Europäisches Patentamt
European Patent Office
Office européen des brevets

(11) EP 3 699 268 A1

(12) EUROPEAN PATENT APPLICATION
published in accordance with Art. 153(4) EPC

(43) Date of publication:
**26.08.2020 Bulletin 2020/35**

(21) Application number: **18869227.1**

(22) Date of filing: **02.09.2018**

(51) Int Cl.:
*C12N 5/10* (2006.01) *C12N 15/867* (2006.01)
*A61K 35/17* (2015.01) *A61P 35/00* (2006.01)
*A61P 31/00* (2006.01) *A61P 37/06* (2006.01)
*A61P 37/08* (2006.01) *A61P 37/02* (2006.01)

(86) International application number:
**PCT/CN2018/103714**

(87) International publication number:
**WO 2019/076149 (25.04.2019 Gazette 2019/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.10.2017 CN 201710983276**

(71) Applicant: **Chongqing Precision Biotech Company Limited**
**Chongqing 400038 (CN)**

(72) Inventors:
- **ZHANG, Wei**
  **Chongqing 400038 (CN)**
- **LI, Wuling**
  **Chongqing 400038 (CN)**
- **ZHANG, Xizhen**
  **Chongqing 400038 (CN)**
- **SHAN, Juanjuan**
  **Chongqing 400038 (CN)**

(74) Representative: **Gill, Siân Victoria et al**
**Venner Shipley LLP**
**200 Aldersgate**
**London EC1A 4HD (GB)**

# (54) UNIVERSAL CAR-T CELL, PREPARATION METHOD THEREFOR AND APPLICATION THEREOF

(57) Provided are a universal CAR-T cell, a preparation method therefor and an application thereof. In the universal CAR-T cell, the functions of T cell antigen receptor (TCR) and major histocompatibility complexes (MHC I and MHC II) in the T cell are inhibited while multi-gene knockout is performed. Genes encoding the TCR comprises TRAC and/or TRBC. Genes encoding the major histocompatibility complexes comprise HLA-A, B2MH and CIITA. The universal CAR-T cell can target relevant markers of specific tumors and inactivate the functions of the TCR and the MHC on the cell surface, can reduce immunological rejection caused by allogeneic cell therapy and safely and effectively remove tumor cells in the diseased human body, and is not affected by the disease or a treatment mode of a patient in use.

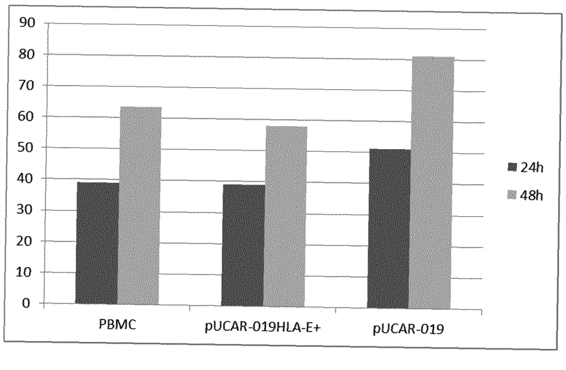


Figure 11



Printed by Jouve, 75001 PARIS (FR)

Description Sequence Listing
<110> CHONGQING PRECISION BIOTECH COMPANY LIMITED
<120> UNIVERSAL CAR-T CELL, PREPARATION METHOD THEREFOR AND APPLICATION THEREOF
<160> 34

<170> PatentIn version 3.3

<210> 1
<211> 449
<212> DNA
<213> Artificial
<220>
<223> B2M-PCR
<400> 1
```
ccttgtcctg attggctggg cacgcgttta atataagtgg aggcgtcgcg ctggcgggca      60
ttcctgaagc tgacagcatt cgggccgaga tgtctcgctc cgtggcctta gctgtgctcg     120
cgctactctc tctttctggc ctggaggcta tccagcgtga gtctctccta ccctcccgct     180
ctggtccttc ctctcccgct ctgcaccctc tgtggccctc gctgtgctct ctcgctccgt     240
gacttccctt ctccaagttc tccttggtgg cccgccgtgg ggctagtcca gggctggatc     300
tcggggaagc ggcggggtgg cctgggagtg gggaaggggg tgcgcacccg ggacgcgcgc     360
tacttgcccc tttcggcggg gagcagggga gacctttggc ctacggcgac gggagggtcg     420
ggacaaagtt tagggcgtcg ataagcgtc                                       449
```

<210> 2
<211> 275
<212>DNA
<213> Artificial
<220>
<223>CIITA2-PCR
<400> 2
```
caccagccct ctttccagaa atttccttct tcatccaagg gacttttcct cccagaaccc      60
gacacagaca ccatcaactg cgaccagttc agcaggctgt tgtgtgacat ggaaggtgat     120
gaagagacca gggaggctta tgccaatatc ggtgaggaag cacctgagcc cagaaaagga     180
caatcaaggg caagagttct ttgctgccac ttgtcaatat cacccattca tcatgagcca     240
cgtcagtccc ctcccacaga aatcattgca agggg                                275
```

<210> 3
<211>229
<212> DNA
<213> Artificial
<220>
<223> TRAC-PCR
<400> 3
```
accctgatcc tcttgtccca cagatatcca gaaccctgac cctgccgtgt accagctgag      60
agactctaaa tccagtgaca agtctgtctg cctattcacc gattttgatt ctcaaacaaa     120
tgtgtcacaa agtaaggatt ctgatgtgta tatcacagac aaaactgtgc tagacatgag     180
gtctatggac ttcaagagca acagtgctgt ggcctggagc aacaaatct                 229
```

<210>4
<211> 78
<212>DNA
<213> Artificial
<220>
<223> HLA-A
<400>4
```
agcaaagtgc gcacccatcc gccgggcctg cgctttagcc cggatgcgga agatggccgc      60
catggcgcgc cgaacccg                                                    78
```

<210>5
<211>20
<212>RNA

<213> sg-B2M-1
<220>
<223>
<400> 5
cgcgagcaca gctaaggcca 20

<210> 6
<211> 20
<212> RNA
<213>
<220>
<223> sg-B2M-2
<400>6
gagtagcgcg agcacagcta 20

<211> 7
<212> RNA
<213> 20
<220>
<223> sg-B2M-3
<400> 7
ggccgagatg tctcgctccg 20

<210>8
<211> 20
<212> RNA
<213>
<220>
<223> sg-HLA-A1
<400> 8
tgacggccat cctcggcgtc 20

<210> 9
<211>20
<212> RNA
<213>
<220>
<223> sg-HLA-A2
<400> 9
gacgccgagg atggccgtca 20

<210>10
<211> 20
<212> RNA
<213>
<220>
<223> sg-HLA-A3
<400> 10
gacggccatc ctcggcgtct 20

<210> 11
<211>20
<212> RNA
<213> Artificial
<220>
<223> sg-HLA-A11-1
<400> 11
cgccatgacg gccatcctcg 20

< 210 >12
<211>20
<212> RNA
<213> Artificial

<220>
<223> sg-HLA-A11-2
<400>12
gcgccatgac ggccatcctc 20

<210> 13
<211>20
<212> RNA
<213> Artificial
<220>
<223> sg-CIITA-1
<400> 13
gctgaactgg tcgcagttga 20

<210> 14
<211>20
<212> RNA
<213> Artificial
<220>
<223> sg-CIITA-2
<400>14
gatattggca taagcctccc 20

<210>15
<211> 20
<212>RNA
<213> Artificial
<220>
<223> sg-CIITA-3
<400> 15
gtcaactgcg accagttcag c 21

<210>16
<211>20
<212> RNA
<213> Artificial
<220>
<223> sg-CIITA-C1
<400>16
ttcctacaca atgcgttgcc 20

<210>17
<211>20
<212> RNA
<213> Artificial
<220>
<223> sg-CIITA-C2
<400>17
agccaggcaa cgcattgtgt 20

<210>18
<211>20
<212>RNA
<213> Artificial
<220>
<223> sg-TRAC-1
<400>18
agagtctctc agctggtaca 20

<210> 19
<211>20
<212>RNA

<213> Artificial
<220>
<223> sg-TRAC-2
<400> 19
tgtgctagac atgaggtcta 20

<210>20
<211> 24
<212> DNA
<213> Artificial
<220>
<223> sg-B2M-3-up primer
<400> 20
caccggccga gatgtctcgc tccg 24

<210>21
<211> 24
<212> DNA
<213> Artificial
<220>
<223> sg-B2M-3-down primer
<400> 21
aaaccggagc gagacatctc ggcc 24

<210>22
<211> 24
<212> DNA
<213> Artificial
<220>
<223> sg-CIITA2-up primer
<400> 22
caccgatatt ggcataagcc tccc 24

<210>23
<211> 24
<212> DNA
<213> Artificial
<220>
<223>sg-CIITA2-down primer
<400> 23
aaacgggagg cttatgccaa tatc 24

<210>24
<211> 24
<212> DNA
<213> Artificial
<220>
<223>sg-TRAC-1-up primer
<400> 24
caccagagtc tctcagctgg taca 24

<210>25
<211> 24
<212> DNA
<213> Artificial
<220>
<223>sg-TRAC-1-down primer
<400> 25
aaactgtacc agctgagaga ctct 24

<210>26
<211> 1381
<212> DNA

<213> Artificial
<220>
<223> PX330A-1X3-B2M-3-CIITA2-TRAC-1
<400> 26
```
gccttttgct ggccttttgc tcacatgtga gggcctattt cccatgattc cttcatattt      60
gcatatacga tacaaggctg ttagagagat aattggaatt aatttgactg taaacacaaa     120
gatattagta caaaatacgt gacgtagaaa gtaataattt cttgggtagt ttgcagtttt     180
aaaattatgt tttaaaatgg actatcatat gcttaccgta acttgaaagt atttcgattt     240
cttggcttta tatatcttgt ggaaaggacg aaacaccggc cgagatgtct cgctccggtt     300
ttagagctag aaatagcaag ttaaaataag gctagtccgt tatcaacttg aaaaagtggc     360
accgagtcgg tgcttttttg ttttagagct agaaatagca agttaaaata aggctagtcc     420
gtttttagcg cgtgcgccaa ttctgcagac aaatggctct agaggcatgt gagggcctat     480
ttcccatgat tccttcatat ttgcatatac gatacaaggc tgttagagag ataattggaa     540
ttaatttgac tgtaaacaca aagatattag tacaaaatac gtgacgtaga aagtaataat     600
ttcttgggta gtttgcagtt ttaaaattat gttttaaaat ggactatcat atgcttaccg     660
taacttgaaa gtatttcgat ttcttggctt tatatatctt gtggaaagga cgaaacaccg     720
atattggcat aagcctcccg ttttagagct agaaatagca agttaaaata aggctagtcc     780
gttatcaact tgaaaaagtg gcaccgagtc ggtgcttttt tgttttagag ctagaaatag     840
caagttaaaa taaggctagt ccgtttttag cgcgtgcgcc aattctgcag acaaatggct     900
ctagaggcat gtgagggcct atttcccatg attccttcat atttgcatat acgatacaag     960
gctgttagag agataattgg aattaatttg actgtaaaca caaagatatt agtacaaaat    1020
acgtgacgta gaaagtaata atttcttggg tagtttgcag ttttaaaatt atgttttaaa    1080
atggactatc atatgcttac cgtaacttga aagtatttcg atttcttggc tttatatatc    1140
ttgtggaaag gacgaaacac cagagtctct cagctggtac agttttagag ctagaaatag    1200
caagttaaaa taaggctagt ccgttatcaa cttgaaaaag tggcaccgag tcggtgcttt    1260
tttgttttag agctagaaat agcaagttaa aataaggcta gtccgttttt agcgcgtgcg    1320
ccaattctgc agacaaatgg ctctagaggt acccgttaca taacttacgg taaatggccc    1380
g                                                                    1381
```

<210>27
<211> 1373
<212> DNA
<213> Artificial
<220>
<223> PX330-A-1X3-B2M-3-TRAC-1-CIITA2
<400> 27
```
gccttttgct ggccttttgc tcacatgtga gggcctattt cccatgattc cttcatattt      60
gcatatacga tacaaggctg ttagagagat aattggaatt aatttgactg taaacacaaa     120
gatattagta caaaatacgt gacgtagaaa gtaataattt cttgggtagt ttgcagtttt     180
aaaattatgt tttaaaatgg actatcatat gcttaccgta acttgaaagt atttcgattt     240
cttggcttta tatatcttgt ggaaaggacg aaacaccggc cgagatgtct cgctccggtt     300
ttagagctag aaatagcaag ttaaaataag gctagtccgt tatcaacttg aaaaagtggc     360
accgagtcgg tgcttttttg ttttagagct agaaatagca agttaaaata aggctagtcc     420
gtttttagcg cgtgcgccaa ttctgcagac aaatggctct agaggcatgt gagggcctat     480
ttcccatgat tccttcatat ttgcatatac gatacaaggc tgttagagag ataattggaa     540
ttaatttgac tgtaaacaca aagatattag tacaaaatac gtgacgtaga aagtaataat     600
ttcttgggta gtttgcagtt ttaaaattat gttttaaaat ggactatcat atgcttaccg     660
taacttgaaa gtatttcgat ttcttggctt tatatatctt gtggaaagga cgaaacacca     720
gagtctctca gctggtacag ttttagagct agaaatagca agttaaaata aggctagtcc     780
gttatcaact tgaaaaagtg gcaccgagtc ggtgcttttt tgttttagag ctagaaatag     840
caagttaaaa taaggctagt ccgtttttag cgcgtgcgcc aattctgcag acaaatggct     900
ctagaggcat gtgagggcct atttcccatg attccttcat atttgcatat acgatacaag     960
gctgttagag agataattgg aattaatttg actgtaaaca caaagatatt agtacaaaat    1020
acgtgacgta gaaagtaata atttcttggg tagtttgcag ttttaaaatt atgttttaaa    1080
atggactatc atatgcttac cgtaacttga aagtatttcg atttcttggc tttatatatc    1140
ttgtggaaag gacgaaacac cgatattggc ataagcctcc cgttttagag ctagaaatag    1200
caagttaaaa taaggctagt ccgttatcaa cttgaaaaag tggcaccgag tcggtgcttt    1260
tttgttttag agctagaaat agcaagttaa aataaggcta gtccgttttt agcgcgtgcg    1320
ccaattctgc agacaaatgg ctctagaggt acccgttaca taacttacgg taa           1373
```

<210>28
<211> 1373

<212> DNA
<213> Artificial
<220>
<223> PX330A-1X3-CIITA2-B2M-3-TRAC-1
<400> 28

```
gccttttgct ggccttttgc tcacatgtga gggcctattt cccatgattc cttcatattt      60
gcatatacga tacaaggctg ttagagagat aattggaatt aatttgactg taaacacaaa     120
gatattagta caaaatacgt gacgtagaaa gtaataattt cttgggtagt ttgcagtttt     180
aaaattatgt tttaaaatgg actatcatat gcttaccgta acttgaaagt atttcgattt     240
cttggcttta tatatcttgt ggaaaggacg aaacaccgat attggcataa gcctcccgtt     300
ttagagctag aaatagcaag ttaaaataag gctagtccgt tatcaacttg aaaaagtggc     360
accgagtcgg tgcttttttg ttttagagct agaaatagca agttaaaata aggctagtcc     420
gtttttagcg cgtgcgccaa ttctgcagac aaatggctct agaggcatgt gagggcctat     480
ttcccatgat tccttcatat ttgcatatac gatacaaggc tgttagagag ataattggaa     540
ttaatttgac tgtaaacaca aagatattag tacaaaatac gtgacgtaga aagtaataat     600
ttcttgggta gtttgcagtt ttaaaattat gttttaaaat ggactatcat atgcttaccg     660
taacttgaaa gtatttcgat ttcttggctt tatatatctt gtggaaagga cgaaacaccg     720
gccgagatgt ctcgctccgg ttttagagct agaaatagca agttaaaata aggctagtcc     780
gttatcaact tgaaaaagtg gcaccgagtc ggtgcttttt tgttttagag ctagaaatag     840
caagttaaaa taaggctagt ccgtttttag cgcgtgcgcc aattctgcag acaaatggct     900
ctagaggcat gtgagggcct atttcccatg attccttcat atttgcatat acgatacaag     960
gctgttagag agataattgg aattaatttg actgtaaaca caaagatatt agtacaaaat    1020
acgtgacgta gaaagtaata atttcttggg tagtttgcag ttttaaaatt atgttttaaa    1080
atggactatc atatgcttac cgtaacttga aagtatttcg atttcttggc tttatatatc    1140
ttgtggaaag gacgaaacac cagagtctct cagctggtac agttttagag ctagaaatag    1200
caagttaaaa taaggctagt ccgttatcaa cttgaaaaag tggcaccgag tcggtgcttt    1260
tttgttttag agctagaaat agcaagttaa aataaggcta gtccgttttt agcgcgtgcg    1320
ccaattctgc agacaaatgg ctctagaggt acccgttaca taacttacgg taa           1373
```

<210>29
<211> 1373
<212> DNA
<213> Artificial
<220>
<223> PX330A-1X3-CIITA2-TRAC-1-B2M-3
<400> 29

```
gccttttgct ggccttttgc tcacatgtga gggcctattt cccatgattc cttcatattt      60
gcatatacga tacaaggctg ttagagagat aattggaatt aatttgactg taaacacaaa     120
gatattagta caaaatacgt gacgtagaaa gtaataattt cttgggtagt ttgcagtttt     180
aaaattatgt tttaaaatgg actatcatat gcttaccgta acttgaaagt atttcgattt     240
cttggcttta tatatcttgt ggaaaggacg aaacaccgat attggcataa gcctcccgtt     300
ttagagctag aaatagcaag ttaaaataag gctagtccgt tatcaacttg aaaaagtggc     360
accgagtcgg tgcttttttg ttttagagct agaaatagca agttaaaata aggctagtcc     420
gtttttagcg cgtgcgccaa ttctgcagac aaatggctct agaggcatgt gagggcctat     480
ttcccatgat tccttcatat ttgcatatac gatacaaggc tgttagagag ataattggaa     540
ttaatttgac tgtaaacaca aagatattag tacaaaatac gtgacgtaga aagtaataat     600
ttcttgggta gtttgcagtt ttaaaattat gttttaaaat ggactatcat atgcttaccg     660
taacttgaaa gtatttcgat ttcttggctt tatatatctt gtggaaagga cgaaacacca     720
gagtctctca gctggtacag ttttagagct agaaatagca agttaaaata aggctagtcc     780
gttatcaact tgaaaaagtg gcaccgagtc ggtgcttttt tgttttagag ctagaaatag     840
caagttaaaa taaggctagt ccgtttttag cgcgtgcgcc aattctgcag acaaatggct     900
ctagaggcat gtgagggcct atttcccatg attccttcat atttgcatat acgatacaag     960
gctgttagag agataattgg aattaatttg actgtaaaca caaagatatt agtacaaaat    1020
acgtgacgta gaaagtaata atttcttggg tagtttgcag ttttaaaatt atgttttaaa    1080
atggactatc atatgcttac cgtaacttga aagtatttcg atttcttggc tttatatatc    1140
ttgtggaaag gacgaaacac cggccgagat gtctcgctcc ggttttagag ctagaaatag    1200
caagttaaaa taaggctagt ccgttatcaa cttgaaaaag tggcaccgag tcggtgcttt    1260
tttgttttag agctagaaat agcaagttaa aataaggcta gtccgttttt agcgcgtgcg    1320
ccaattctgc agacaaatgg ctctagaggt acccgttaca taacttacgg taa           1373
```

<210>30

<211> 1373
<212> DNA
<213> Artificial
<220>
<223> PX330A-1X3-TRAC-1-B2M-3-CIITA2
<400> 30

```
gccttttgct ggccttttgc tcacatgtga gggcctattt cccatgattc cttcatattt      60
gcatatacga tacaaggctg ttagagagat aattggaatt aatttgactg taaacacaaa     120
gatattagta caaaatacgt gacgtagaaa gtaataattt cttgggtagt ttgcagtttt     180
aaaattatgt tttaaaatgg actatcatat gcttaccgta acttgaaagt atttcgattt     240
cttggcttta tatatcttgt ggaaaggacg aaacaccaga gtctctcagc tggtacagtt     300
ttagagctag aaatagcaag ttaaaataag gctagtccgt tatcaacttg aaaaagtggc     360
accgagtcgg tgcttttttg ttttagagct agaaatagca agttaaaata aggctagtcc     420
gtttttagcg cgtgcgccaa ttctgcagac aaatggctct agaggcatgt gagggcctat     480
ttcccatgat tccttcatat ttgcatatac gatacaaggc tgttagagag ataattggaa     540
ttaatttgac tgtaaacaca aagatattag tacaaaatac gtgacgtaga aagtaataat     600
ttcttgggta gtttgcagtt ttaaaattat gttttaaaat ggactatcat atgcttaccg     660
taacttgaaa gtatttcgat ttcttggctt tatatatctt gtggaaagga cgaaacaccg     720
gccgagatgt ctcgctccgg ttttagagct agaaatagca agttaaaata aggctagtcc     780
gttatcaact tgaaaaagtg gcaccgagtc ggtgcttttt tgttttagag ctagaaatag     840
caagttaaaa taaggctagt ccgtttttag cgcgtgcgcc aattctgcag acaaatggct     900
ctagaggcat gtgagggcct atttcccatg attccttcat atttgcatat acgatacaag     960
gctgttagag agataattgg aattaatttg actgtaaaca caaagatatt agtacaaaat    1020
acgtgacgta gaaagtaata atttcttggg tagtttgcag ttttaaaatt atgttttaaa    1080
atggactatc atatgcttac cgtaacttga aagtatttcg atttcttggc tttatatatc    1140
ttgtggaaag gacgaaacac cgatattggc ataagcctcc cgttttagag ctagaaatag    1200
caagttaaaa taaggctagt ccgttatcaa cttgaaaaag tggcaccgag tcggtgcttt    1260
tttgttttag agctagaaat agcaagttaa aataaggcta gtccgttttt agcgcgtgcg    1320
ccaattctgc agacaaatgg ctctagaggt acccgttaca taacttacgg taa           1373
```

<210>31
<211> 1374
<212> DNA
<213> Artificial
<220>
<223> PX330A-1X3-TRAC-1-CIITA2-B2M-3
<400> 31

```
gccttttgct ggccttttgc tcacatgtga gggcctattt cccatgattc cttcatattt      60
gcatatacga tacaaggctg ttagagagat aattggaatt aatttgactg taaacacaaa     120
gatattagta caaaatacgt gacgtagaaa gtaataattt cttgggtagt ttgcagtttt     180
aaaattatgt tttaaaatgg actatcatat gcttaccgta acttgaaagt atttcgattt     240
cttggcttta tatatcttgt ggaaaggacg aaacaccaga gtctctcagc tggtacagtt     300
ttagagctag aaatagcaag ttaaaataag gctagtccgt tatcaacttg aaaaagtggc     360
accgagtcgg tgcttttttg ttttagagct agaaatagca agttaaaata aggctagtcc     420
gtttttagcg cgtgcgccaa ttctgcagac aaatggctct agaggcatgt gagggcctat     480
ttcccatgat tccttcatat ttgcatatac gatacaaggc tgttagagag ataattggaa     540
ttaatttgac tgtaaacaca aagatattag tacaaaatac gtgacgtaga aagtaataat     600
ttcttgggta gtttgcagtt ttaaaattat gttttaaaat ggactatcat atgcttaccg     660
taacttgaaa gtatttcgat ttcttggctt tatatatctt gtggaaagga cgaaacaccg     720
atattggcat aagcctcccg ttttagagct agaaatagca agttaaaata aggctagtcc     780
gttatcaact tgaaaaagtg gcaccgagtc ggtgcttttt tgttttagag ctagaaatag     840
caagttaaaa taaggctagt ccgtttttag cgcgtgcgcc aattctgcag acaaatggct     900
ctagaggcat gtgagggcct atttcccatg attccttcat atttgcatat acgatacaag     960
gctgttagag agataattgg aattaatttg actgtaaaca caaagatatt agtacaaaat    1020
acgtgacgta gaaagtaata atttcttggg tagtttgcag ttttaaaatt atgttttaaa    1080
atggactatc atatgcttac cgtaacttga aagtatttcg atttcttggc tttatatatc    1140
ttgtggaaag gacgaaacac cggccgagat gtctcgctcc ggttttagag ctagaaatag    1200
caagttaaaa taaggctagt ccgttatcaa cttgaaaaag tggcaccgag tcggtgcttt    1260
tttgttttag agctagaaat agcaagttaa aataaggcta gtccgttttt agcgcgtgcg    1320
ccaattctgc agacaaatgg ctctagaggt acccgttaca taacttacgg taaa          1374
```

<210>32
<211> 1329
<212> DNA
<213> Artificial
<220>
<223> HLA-F
<400> 32

```
atggcgcccc gaagcctcct cctgctgctc tcagggggccc tggccctgac cgatacttgg      60
gcgggctccc actccttgag gtatttcagc accgctgtgt cgcggcccgg ccgcggggag     120
ccccgctaca tcgccgtgga gtacgtagac gacacgcaat tcctgcggtt cgacagcgac     180
gccgcgattc cgaggatgga gccgcgggag ccgtgggtgg agcaagaggg gccgcagtat     240
tgggagtgga ccacagggta cgccaaggcc aacgcacaga ctgaccgagt ggccctgagg     300
aacctgctcc gccgctacaa ccagagcgag gctgggtctc acaccctcca gggaatgaat     360
ggctgcgaca tggggcccga cggacgcctc ctccgcgggt atcaccagca cgcgtacgac     420
ggcaaggatt acatctccct gaacgaggac ctgcgctcct ggaccgcggc ggacaccgtg     480
gctcagatca cccagcgctt ctatgaggca gaggaatatg cagaggagtt caggacctac     540
ctggagggcg agtgcctgga gttgctccgc agatacttgg agaatgggaa ggagacgcta     600
cagcgcgcag atcctccaaa ggcacacgtt gcccaccacc ccatctctga ccatgaggcc     660
accctgaggt gctgggccct gggcttctac cctgcggaga tcacgctgac ctggcagcgg     720
gatggggagg aacagaccca ggacacagag cttgtggaga ccaggcctgc aggggatgga     780
accttccaga agtgggccgc tgtggtggtg cctcctggag aggaacagag atacacatgc     840
catgtgcagc acgaggggct gccccagccc ctcatcctga gatgggagca gtctccccag     900
cccaccatcc ccatcgtggg catcgttgct ggccttgttg tccttggagc tgtggtcact     960
ggagctgtgg tcgctgctgt gatgtggagg aagaagagct cagatagaaa cagagggagc    1020
tactctcagg ctgcagccta ctcagtggtc agcggaaact tgatgataac atggtggtca    1080
agcttatttc tcctgggggt gctcttccaa ggatatttgg gctgcctccg gagtcacagt    1140
gtcttgggcc gccggaaggt gggtgacatg tggatcttgt ttttttgtg gctgtggaca     1200
tctttcaaca ctgccttctt ggccttgcaa agccttcgct ttggcttcgg ctttaggagg    1260
ggcaggagct tccttcttcg ttcttggcac catcttatga aagggtcca gattaagatt     1320
tttgactga                                                            1329
```

<210>33
<211> 1077
<212> DNA
<213> Artificial
<220>
<223> HLA-E
<400> 33

```
atggtagatg gaaccctcct tttactcctc tcggaggccc tggcccttac ccagacctgg      60
gcgggctccc actccttgaa gtatttccac acttccgtgt cccggcccgg ccgcggggag     120
ccccgcttca tctctgtggg ctacgtggac gacacccagt tcgtgcgctt cgacaacgac     180
gccgcgagtc cgaggatggt gccgcgggcg ccgtggatgg agcaggaggg gtcagagtat     240
tgggaccggg agacacggag cgccagggac accgcacaga ttttccgagt gaacctgcgg     300
acgctgcgcg gctactacaa tcagagcgag gccgggtctc acaccctgca gtggatgcat     360
ggctgcgagc tggggcccga caggcgcttc ctccgcgggt atgaacagtt cgcctacgac     420
ggcaaggatt atctcaccct gaatgaggac ctgcgctcct ggaccgcggt ggacacggcg     480
gctcagatct ccgagcaaaa gtcaaatgat gcctctgagg cggagcacca gagagcctac     540
ctggaagaca catgcgtgga gtggctccac aaatacctgg agaaggggaa ggagacgctg     600
cttcacctgg agcccccaaa gacacacgtg actcaccacc ccatctctga ccatgaggcc     660
accctgaggt gctgggccct gggcttctac cctgcggaga tcacactgac ctggcagcag     720
gatggggagg gccataccca ggacacggag ctcgtggaga ccaggcctgc aggggatgga     780
accttccaga agtgggcagc tgtggtggtg ccttctggag aggagcagag atacacgtgc     840
catgtgcagc atgaggggct acccgagccc gtcaccctga gatggaagcc ggcttcccag     900
cccaccatcc ccatcgtggg catcattgct ggcctggttc tccttggatc tgtggtctct     960
ggagctgtgg ttgctgctgt gatatggagg aagaagagct caggtggaaa aggagggagc    1020
tactctaagg ctgagtggag cgacagtgcc caggggtctg agtctcacag cttgtaa       1077
```

<210>34
<211> 358
<212> Protein
<213> Artificial
<220>
<223> HLA-E
<400> 34

Met Val Asp Gly Thr Leu Leu Leu Leu Leu Ser Glu Ala Leu Ala
5 10
15
Leu Thr Gln Thr Trp Ala Gly Ser His Ser Leu Lys Tyr Phe His
20 25
30
Thr Ser Val Ser Arg Pro Gly Arg Gly Glu Pro Arg Phe Ile Ser
35 40
45
Val Gly Tyr Val Asp Asp Thr Gln Phe Val Arg Phe Asp Asn Asp
50 55
60
Ala Ala Ser Pro Arg Met Val Pro Arg Ala Pro Trp Met Glu Gln
65 70
75
Glu Gly Ser Glu Tyr Trp Asp Arg Glu Thr Arg Ser Ala Arg Asp
80 85
90
Thr Ala Gln Ile Phe Arg Val Asn Leu Arg Thr Leu Arg Gly Tyr
95 100
105
Tyr Asn Gln Ser Glu Ala Gly Ser His Thr Leu Gln Trp Met His
110 115
120
Gly Cys Glu Leu Gly Pro Asp Arg Arg Phe Leu Arg Gly Tyr Glu
125 130
135
Gln Phe Ala Tyr Asp Gly Lys Asp Tyr Leu Thr Leu Asn Glu Asp
140 145
150
Leu Arg Ser Trp Thr Ala Val Asp Thr Ala Ala Gln Ile Ser Glu
155 160
165
Gln Lys Ser Asn Asp Ala Ser Glu Ala Glu His Gln Arg Ala Tyr
170 175
180
Leu Glu Asp Thr Cys Val Glu Trp Leu His Lys Tyr Leu Glu Lys
185 190
195
Gly Lys Glu Thr Leu Leu His Leu Glu Pro Pro Lys Thr His Val
200 205
210
Thr His His Pro Ile Ser Asp His Glu Ala Thr Leu Arg Cys Trp
215 220
225
Ala Leu Gly Phe Tyr Pro Ala Glu Ile Thr Leu Thr Trp Gln Gln
230 235
240
Asp Gly Glu Gly His Thr Gln Asp Thr Glu Leu Val Glu Thr Arg
245 250
255
Pro Ala Gly Asp Gly Thr Phe Gln Lys Trp Ala Ala Val Val Val
260 265
270
Pro Ser Gly Glu Glu Gln Arg Tyr Thr Cys His Val Gln His Glu
275 280
285
Gly Leu Pro Glu Pro Val Thr Leu Arg Trp Lys Pro Ala Ser Gln
290 295
300
Pro Thr Ile Pro Ile Val Gly Ile Ile Ala Gly Leu Val Leu Leu
305 310
315

```
Gly Ser Val Val Ser Gly Ala Val Val Ala Ala Val Ile Trp Arg
                                320                                 325
330
Lys Lys Ser Ser Gly Gly Lys Gly Gly Ser Tyr Ser Lys Ala Glu
                                335                                 340
345
Trp Ser Asp Ser Ala Gln Gly Ser Glu Ser His Ser Leu
                                350                                 355
```

## Description

### TECHNICAL FIELD

The present invention relates to immunotherapy field, and more particularly relates to the universal CAR-T cells and preparation method and application thereof. Furthermore, it relates to the universal CAR-T cells and the preparation method thereof and application in medicine.

### BACKGROUND

Adoptive cell therapy (ACT) is a kind of biological therapy technology, which can amplify autoimmune cells (mainly T cells) in vitro and then transfer them back into cancer patients to achieve the therapeutic purpose. It is considered as the fourth treatment after surgery, radiotherapy and chemotherapy, and is widely used in clinical treatment.
The chimeric antigen receptor, also known as CAR, is an artificial receptor that mimics the function of TCR. When the antigen (receptor) on the surface of the tumor cells binds to the antibody (ligand) of the CAR, the signal is transmitted to the intracellular region through the hinge region and the transmembrane region, and the intracellular signal domain converts the signal into an activation signal and the effector cells are activated. The effector cells can kill tumor cells by secreting perforin or producing cytokines, and the effector cells themselves are amplificated to further expand the immune killing effect.
In recent years, with the significant effect of CAR-modified T cells on the treatment of hematological tumors, the demand for adoptive cell therapy is increasing. However, current adoptive cell therapy is based on autologous cells reinfusion therapy. This therapy requires a certain amount of peripheral blood from patients, followed by peripheral blood mononuclear cell separation and T lymphocyte separation, then the T lymphocytes are modified and expanded in vitro, and proliferate the modified T lymphocytes to a certain number and then return to patients to achieve a certain therapeutic effect. However, most patients, especially tumor patients, have undergone other treatments before the CAR-T cell adoptive therapy, such as chemotherapy and radiotherapy, which results in fewer T lymphocytes being obtained, even the expansion of T lymphocytes from patients in vitro are difficult and the T cells of the patient's own source may also have functional attenuation or loss due to drugs, and the difficulty of genetic modification of T cells is increased and the therapeutic effect and safety are not easy to control. These obstacles affect the application and development of CAR-T cell therapy and clinical treatment of tumors.
Allogeneic cells refer to cells belonging to the same species but genetically different individuals. Although more T lymphocytes can be separated from healthy human peripheral blood, and the positive rate of CAR infection is higher and the viability and function are superior to the patient-derived T lymphocytes during allogeneic cell therapy, the allogeneic cell therapy faces many obstacles due to immunogenetic differences between donor and recipient. On one hand, immune-active donor T lymphocytes enter the recipient patient and proliferate to certain extent will cause a graft versus host response (GVHD) as the normal cells or tissues of the recipient patient are mistaken as target to attack. On the other hand, the normal immune system, which is the recipient of allogeneic cells, may clear it and produce a host versus graft response (HVGR) that may affect the therapeutic effect. Currently, immunosuppressive drugs are mainly used to inhibit receptor immune system activity before treatment to against HVGR, but immunosuppressive drugs may affect the efficacy of reinfusion cells in adoptive cell therapy, and HLA matching is mainly used in medical treatment to against GVHD, however, the HLA matching is time consuming and costly, and the success rate is low.
Therefore, it is necessary to develop a safe and effective universal CAR-T cell which can reduce the immune rejection caused by allogeneic cell therapy.

### SUMMARY

In view of this, the present invention is directed to one type of universal CAR-T cell, which can target a specific tumor-associated marker and inactivate TCR and MHC on the cell surface, and can reduce the immune rejection caused by allogeneic cell therapy remove tumor cells in patients safely and effectively.
To achieve the above object, the technical solution of the present invention is shown below.
A universal CAR-T cell, wherein both T Cell Antigen Receptor (TCR) and Major Histocompatibility Complex (MHC I, MHC II) functions of the universal CAR-T cell have been inhibited by polygenic knockout; genes encoding the TCR include TRAC and/or TRBC; genes encoding the Major Histocompatibility Complex include HLA-A, B2M and CIITA.
The CAR-T treatment of autologous cells requires preparation of patients' own T lymphocytes separated from blood. On one hand, due to the patient's own condition and the different T lymphocytes status, there are many influential factors affecting the CAR-T standardized production process and safety. On the other hand, some patient's autologous T lymphocytes have insufficient activity and quantity after chemotherapy, or are affected by the tumor environment, resulting in limited T lymphocyte activity and proliferative capacity. Such cells are difficult to prepare for CAR-T, and the safety and efficacy of treatment are affected; or in the process of CAR-T cell preparation, if the prepared cells cannot be timely returned to patients, the therapeutic effect will also be affected, and even autologous CAR-T cells adoptive therapy is not available for some tumor patients affected by autologous T lymphocyte status. Hence, the development of universal CAR-T or universal T lymphocyte therapy for allogeneic therapy is imminent.

We simultaneously knocked out the genes TRAC, B2M and CIITA or TRAC, HLA-A and CIITA, and the TCR and MHC I and MHC II were all inactivated, and the obtained universal CAR-T cells were highly safe. On the other hand, considering that NK cells in vivo recognize "autologous and non-autologous" through MHCI and attack the identified "non- autologous", resulting in the NK cells in patients recognizing and removing the universal CAR-T,the survival time of universal CAR-T in patients is short and the efficacy is poor, we made some improvements: simultaneously expressing HLA-E or HLA-F can recognize molecules on the surface of NK cells, making NK cells to recognize the cells to be "autologous" cells and do not attack.

Further, the TRAC, the B2M and the CIITA genes have been knocked out from said universal CAR-T cell, and the HLA-E or the HLA-F is expressed in the universal CAR-T cell.

Host versus graft reaction (HVGR) and graft versus host reaction (GVHR) are one of the largest problems in CAR-T treatment except for Cytokine release syndrome (CRS). HVGR and GVHR related genes include TCR and MHC I and MHC II related genes, and a single TRAC gene is a complete functional TCR complex formed by the gene encoding a TCRachain and two TRBC genes encoding TCRβ. Knocking out TRAC is the optimal solution to inactivate TCR. B2M and CIITA are MHC I and MHC II related genes respectively. Returning T lymphocytes with the above 3 genes knocked out to allogeneic patients do not cause graft versus host response (GVHD).

However, when the said universal CAR-T cells were tested, the inventors found that, the universal CAR-T cells were quickly removed by allogeneic NK cells and had a short survival time. Probably because B2M interference will cause complete inactivating of all genes from the MHCI family. Although this can completely eliminate GVHD, the expression of MHC is a key molecule for NK cells to recognize "autologous cells", and the MHC I complete inactivation makes NK cells to remove the reinfusion cells as "allogeneic", affecting the efficacy of universal CAR-T cells against tumors.

Co-expressing conserved genes in the human genome, such as HLA-E or HLA-F, in the MHC I subtype while infecting CAR, and then knock out the TRAC, B2M and CIITA genes using CRISPR/Cas9 to re-label the universal CAR-T cells as 'autologous', to achieve the goal of attenuating GVDH without causing NK cell attack.

Preferably, knock out the TRAC, B2M and CIITA genes from the said universal CAR-T cells while also express HLA-E or HLA-F.

Preferably, knock out the TRAC, B2M and CIITA genes from the said universal CAR-T cells while also express HLA-E.

The applicant team had previously performed HLA typing tests on blood from different healthy donors. HLA is a highly polymorphic alloantigen controlled by the human major histocompatibility complex (MHC) gene cluster, HLA is equivalent to the above said MHC molecules. It was found that HLA-A was mainly concentrated in 8 types in the test samples, and the 8 types were A*02, A*11, A*24, A*30, A*33, A*03, A*01 and A*26 respectively. When PCR amplification was performed for these 8 types, to obtained PCR fragments for gRNA design, it was found that 7 of them can design gRNA in the same region. Furthermore, the inventor team used the bioinformatics to analyze different HLA-A transcript ratios by big data, and found that the frequency of the eight genotypes was the highest among Chinese with more than 90% of the total genotypes. The 8 most frequently genotyped gRNA design region gene sequences are shown in SEQ ID NO:4, and the gRNA sequences are shown in SEQ ID NO:8-12.

HLA-A knockout makes HLA inactivation but does not affect the function of human conserved HLA-E and HLA-F (human conserved HLA molecules have the same phenotype in all humans), choose HLA-A gene to replace the B2M gene, and knock out TRAC, HLA-A and CIITA to achieve universal CAR-T cells that neither caused GVHD nor NK specific recognition.

Furthermore, the TRAC, the HLA-A or the B2M, the CIITA genes have been knocked out from the universal CAR-T cell.

The second objective of the present invention is directed to provide a method for producing the universal CAR-T cell, wherein the method comprises:

1) obtain activated T cells.

2) transfect the activated T cells of step 1) with lentiviral vector expressing CAR or co-expressing CAR and HLA-E or HLA-F, wherein the CAR is encoded to target different tumor-related molecules; and obtain CAR-T cells targeting the different tumor-related molecules.

3) produce polygenic knockout of the CAR-T cells of step 2) by TALEN, zinc finger or CRISPR/Cas9 system methods to obtain the universal CAR-T cells, wherein knockout genes include TRAC, HLA-A and CIITA or TRAC, B2M and CIITA.

The said universal CAR-T cells are capable of targeting specific tumor related markers and inactivating TCR and MHC on the cell surface, but the preparation method needs to overcome two technical obstacles: 1. the feasibility of simultaneously achieve polygenic knockout and polygenic expression in T lymphocytes. 2. Whether the obtained universal CAR-T or T lymphocytes can safely and effectively target tumor cells with specific targets.

Polygenic knockout studies are basically done on T lymphocytes. It is technically difficult to perform polygenic knockout on CAR-T to implement gene knockout together with CAR-T expression. The general method of gene knockout is described below. CRISPR / cas9 vector and CAR vector with sgRNA of the target gene to be knocked out are repeatedly infected by virus vector, and the cell activity is decreased due to multiple virus infections, while polygenic gene knockout results in extremely low transfection efficiency when the vector is too large with virus or transfection reagent, and only electrotransfection can achieve the purpose of the transfection vector, but the applicant team found that the cell state after electrotransfection is poor and needs a certain recovery period, but T-lymphocytes cannot survive in vitro for a long time (even if the activation reagent is used for stimulation, the maximum survival time in vitro is 2-3 weeks). The infection efficiency of car is low and the cell activity is poor after electrotransfection.

Therefore, the applicant optimized the infection conditions to prepare CAR-T cells by CAR infection 24 hours after the activation of T cells. After 24 hours of culture, implement electrotransfection. The CRISPR/Cas9 vector with sgRNA of the target gene to be knocked out was transfected to construct universal CAR-T cells. The knockout efficiency of the method is high, the obtained universal CAR-T cell allogeneic reinfusion is safe, and the positive rate of the infected CAR is stable and has good killing activity and specificity.

For therapeutic purposes, CAR-T or T lymphocytes (T cells) used for adoptive transfer or allogeneic reinfusion are prepared from peripheral blood-separated T cells, but the T cells separated from peripheral blood are mature T cells with limited time to maintain proliferation and effective function in vitro, therefore the genetically modified universal cells need to be rapidly expanded in vitro to the required number of treatments and then returned to the patient. Through a lot of experiments, the inventors got the best way to build universal CAR-Tor T cells by combining virus infection and electrotransfection.1) Polygenic expression using viral infection. 2) Polygenic interference using CRISPR/Cas9 technology. However, at present, the commonly used virus-infected expression genes are relatively mature for single-gene infection technology. Polygenic infections are often inefficient, and Polygenicknockout is involved at the same time. Although CRISPR/Cas9 technology can achieve stable knockout of target genes, knockout efficiency of polygenic interference by virus infection is low, and the use of electrotransfection has a great influence on cell activity and the optimal electrotransfection conditions require a large number of exploration experiments.

In step 2), CAR-T cells are first obtained by infecting T lymphocytes with CAR encoding specific tumor targets and viruses co-expressed with HLA-E or HLA-F, and further polygenic knockout is performed.

Furthermore, the target molecules recognized by the CAR in step 2) comprise one of CD19, PSCA, CD123, CD20, CEA (carcinoembryonic antigen), FAP, CD133, EGFR, EGFRVIII, BCMA, PSMA, Her2, CA125, EphA2, C-met, L1CAM, VEGFR, CS1, ROR1, EC, NY-ESO-1, MUC1, MUC16, mesothelin, LewisY, GPC3, GD2, EPG, DLL3, CD99, 5T4, CD22, CD30, CD33, CD138, CD171.

Furthermore, the polygenic knockout in step 3) is produced using the CRISPR/Cas9 system method.

Furthermore, the CRISPR/Cas9 system consists of gRNA and Cas9 nuclease or endonuclease.

Furthermore, electrotransfection method is preferably chosen for polygenic knockout by the CRISPR/Cas9 system, and gRNA of the knockout genes is constructed in a vector, and the knockout genes are knocked out by the electrotransfection method.

Furthermore, the gRNA sequences targeting the B2M, HLA-A, CIITA and TRAC genes are shown in SEQ ID NO: 5-19.

Furthermore, the gRNA sequences targeting the TRAC, B2M and CIITA or HLA-A, CIITA and TRAC genes are constructed on vector PX330A.

The expressed HLA-E gene sequence is shown in SEQ ID NO:33, the protein sequence is shown in SEQ ID NO:34, and the expressed HLA-F gene sequence is shown in SEQ ID NO: 32.

How to choose the knockout method to achieve high transfection efficiency and cell survival rate is a difficult problem. The applicant found that the method of multiple transfection to achieve polygenic knockout would seriously affect cell activity through multiple experiments. The applicant designed gRNA targeting B2M, CIITA and TRAC or HLA-A, CIITA and TRAC genes to be constructedin the same vector, and then transfected by electrotransfectionfor gene knockout. In the absence of more effective prompting techniques, the applicant explored the optimization of the electrotransfection conditions through a large number of experiments: the difference in the electrotransfection buffer, voltage, pulse size and number of electric shocks used in electrotransfection will affect the cell survival rate and the electrotransfection efficiency.

Preferably, 293T cells are transfected with GFP, and the electrotransfection conditions: 48 hours after PBMC or T cells activation, the voltage is 1000V and the pulse width is 35ms, electrotransfection are conducted twice, the total number of cells is 2 × $10^5$, and the cells are cultured in fresh medium containing IL-2 after electrotransfection.

The third objective of the present invention is directed to CRISPR/Cas9 gene knockout expression vector obtained from the method of Claim 4, wherein the expression vector comprises gene sequences shown in SEQ ID NO: 26-31.

The present invention is also directed to use of the universal CAR-T cells in a medicaments for allogeneic treatment.

The universal type cells can be obtained by the method described above, and the universal CAR-T cells which are targeting specific tumors and simultaneously inactivating TCR and MHC I and MHC II molecules on the cell surface, or the universal T lymphocytes simultaneously inactivating TCR and MHC I and MHC II molecules on the cell surface, which can improve the safety and efficacy of CAR-T cells in tumor treatment.

The present invention is also directed to use of the universal CAR-T cells in the manufacture of a medicament for malignant tumor or infectious disease.

Furthermore, cells or tissues of the malignant tumor or the infectious disease are capable of expressing one of CD19, PSCA, CD123, CD20, CEA (carcinoembryonic antigen), FAP, CD133, EGFR, EGFRVIII, BCMA, PSMA, Her2, CA125, EphA2, C-met, L1CAM, VEGFR, CS1, ROR1, EC, NY-ESO-1, MUC1, MUC16, mesothelin, LewisY, GPC3, GD2, EPG, DLL3, CD99, 5T4, CD22, CD30, CD33, CD138, CD171. The universal CAR-T comprises UCAR-19 and UCAR-PSCA.

Furthermore, the malignant tumor or the infectious disease comprise: hematological tumor, solid tumor, immune rejection caused by allogeneic transplantation, and autoimmune diseases such as allergic reaction or systemic lupus erythematosus.

The present invention is also directed to a universal T cell in the manufacture of a medicament for allogeneic treatment, wherein functions of T cell Antigen Receptor (TCR) and Major Histocompatibility Complex (MHC I, MHC II) are simultaneously inhibited in the universal T cell; and chimeric antigen receptors that recognize the malignant tumors or the infected cells are expressed.

Furthermore, the genes encoding the TCR include TRAC and/or TRBC, and the genes encoding the Major Histocompatibility Complex include HLA, B2MH and CIITA.

Further, the universal T cell expresses HLA-E or HLA-F; and TRAC, HLA-A and CIITA genes or TRAC, B2M and CIITA genes have been knocked out.

This universal T cell can be used for allogeneic treatment.

The present invention is also directed to use of the universal T cells in the manufacture of medicament for malignant tumor or infectious diseases.

Furthermore, the malignant tumor or the infectious disease comprises: hematological tumor, solid tumor, immune rejection caused by allogeneic transplantation, and autoimmune diseases such as allergic reaction or systemic lupus erythematosus.

Among the genes related to HVGR and GVHR, the applicant designed different research protocols by gene editing technology to conduct gene editing on T lymphocytes from healthy donors, and finally obtained allogeneic universal T cells with low rejection, long survival time in patients, high safety, which made allogeneic cell therapy more widely used and promote the standardized production of cell therapy. The applicant further uses the universal T lymphocytes to successfully carry out CAR-T transformation to obtain the universal allogeneic CAR-T cells, further improving the safety and efficacy of CAR-T treatment.

In general, the universal T cells and universal CAR-T cells of the present invention can be applied to the treatment of malignant tumors or infectious diseases by means of allogeneic reinfusion, and the low rejection, long survival time in patient, and high safety help with the therapeutic effect and safety problems for the majority of patients, especially cancer patients, who have undergone chemotherapy, and radiotherapy leading to fewer autologous T cells, even T cell function attenuation or loss due to disease, and provide help for the promotion and development of CAR-T cell therapy.

**Advantages of the present invention:**

1) The method for preparing universal CAR-T cells provided by the present invention can obtain universal CAR-T cells with high CAR expression rate and simultaneously inactivation of TCR and MHC. The said universal CAR-T cells have low rejection when applied to the treatment of malignant tumors or infectious diseases and have high efficacy and safety in patients.

2) The universal CAR-T cells provided by the present invention are applied to the treatment of allogeneic adoptive cells without being affected by the patients' own condition or treatment mode, and the required universal car-t can be prepared at any time to give patients treatment at the best time to ensure the effectiveness of treatment.

3) The universal T cells provided by the present invention can be applied to immunotherapy T cells of allogeneic reinfusion, and the said universal T cells are convenient for general preparation and have low rejection after allogeneic reinfusion.

**BRIEF DESCRIPTION**

Figure 1 shows the results of HLA-A typing frequency detection in Chinese population.

Figure 2 shows the design of knocking out TCR and MHC I and MHC II molecular related gene vectors using the CRISPR/Cas9 system.

Figure 3 shows the designed gRNA targeting site detection.

Figure 4 shows the detection of B2M, CIITA and TRAC expression on CAR-T cell surface after knocking out by CRISPR/Cas9 system.

Figure 5 shows the CAR expression detection.

Figure 6 is the detection of simultaneous inactivation efficiency of TCR and MHC I and MHC II molecules on CAR-T cells after knocking out by CRISPR/Cas9 system and the triple-negative CAR-T phenotype after sorting.

Figure 7 shows the CRISPR/Cas9 off-target detection.

Figure 8 shows the detection of reactivity of triple-negative CAR-T cells to allogeneic NK cells.

Figure 9 shows the detection of the universal CAR-T HLA-E expression.

Figure 10 shows the detection of the capability of universal CAR-T to kill tumor cells.

Figure 11 shows the detection of reactivity of universal CAR-T cells to allogeneic NK cells.

**EMBODIMENTS**

Hereinafter, preferred embodiments of the present invention will be described in detail with reference to the accompanying drawings. Wherever there are no specific experimental conditions specified, the experimental methods in general follow the conventional conditions, such as those described in the Molecular Cloning Experiment Guide (third edition, J. Sambroke et al.), or those recommended by the manufacturers. The described embodiments are intended to better illustrate the contents of the invention, but the contents of the invention are not limited to the embodiments. Therefore, any non-essential improvement and adjustment to the embodiments implemented by the person skilled in the art according to the above-mentioned inventions shall still fall within the scope of protection of the present invention.

**Embodiment 1: HLA-A typing frequency detection in Chinese population.**

1. SBT (Sequenced Based Typing) detection:

This method relates to the HLA high resolution typing technique.
The EDTA anticoagulation tube (Routine blood test tube) is used to take 4 ml of venous blood from the examinee, and temporarily store at 4°C and store at -20°C for a long time, then send to BGI Co., LTD for HLA typing detection.

2. Bioinformatics methods: refer to Boegel, Sebastian, Valesca et al. (2013)

The HLA high-resolution typing (MHC-I and MHC-II, 4 positions) of the sample and the corresponding expression values and p-values are calculated directly from the raw data of RNA-Seq sequencing (fastq format) using seq2HLA software.

The experimental results are shown in Figure 1. 8 HLA-A types with the highest frequency in Chinese are obtained, which are A*02, A*11A*24, A*30, A*33, A*03, A*01 and A*26 respectively. gRNA molecules are designed for these 8 subtypes, and the gRNA sequences are shown in SEQ ID NOs: 8-12.

**Embodiment 2: Construction of gene vectors related with knockout of TCR and MHC I and MHC II molecules**

1. sgRNA molecular design:

Identify genes related with TCR and MHC I and MHC II molecules: the functions of genes of B2M, CIITA and TRAC are closely corelated to the functions of related genes of TCR and MHC I and MHC II molecules, and the genes of B2M, CIITA, and TRAC are confirmed to be knocked out. Obtain the B2M, CIITA and TRAC gene sequence numbers and the CDs sequence on NCBI, the B2M sequence number is AH002619.2, CIITA ID in NCBI is 4261, TRAC ID in NCBI is 28755, and these gene sequences are shown in SEQ ID No: 1-3. Design gRNA corresponding to the targeted area of the said sequences of SEQ ID No: 1-3 in CDS area, and the gRNA sequences are shown in Table 1.

Table 1: gRNA sequences

| Gene Name | Sequence Name | Sequence |
|---|---|---|
| HLA-A | sg-B2M-1 | CGCGAGCACAGCTAAGGCCA |
| | sg-B2M-2 | GAGTAGCGCGAGCACAGCTA |
| | sg-B2M-3 | GGCCGAGATGTCTCGCTCCG |
| | sg-HLA-A1 | TGACGGCCATCCTCGGCGTC |
| | sg-HLA-A2 | GACGCCGAGGATGGCCGTCA |
| | sg-HLA-A3 | GACGGCCATCCTCGGCGTCT |
| | sg-HLA-A11-1 | CGCCATGACGGCCATCCTCG |
| | sg-HLA-A11-2 | GCGCCATGACGGCCATCCTC |
| CIITA (HLA-II molecular transcription factors) | sg-CIITA-1 | GCTGAACTGGTCGCAGTTGA |
| | sg-CIITA-2 | GATATTGGCATAAGCCTCCC |
| | sg-CIITA-3 | GTCAACTGCGACCAGTTCAGC |
| | sg-CIITA-C1 | TTCCTACACAATGCGTTGCC |
| | sg-CIITA-C2 | AGCCAGGCAACGCATTGTGT |
| TCR | sg-TRAC-1 | AGAGTCTCTCAGCTGGTACA |
| | sg-TRAC-2 | TGTGCTAGACATGAGGTCTA |
| | sg-TRBC | TCTCCGAGAGCCCGTAGAAC |
| | sg-TCR | GAGAATCAAAATCGGTGAAT |

2. Construction of expression vector with CRISPR/Cas9 gene knockout

Design the following primers, and synthesized by GenScript Co., Ltd. The specific primers are shown below:

Primer sg-B2M-3-up: 5’-CACCGGCCGAGATGTCTCGCTCCG-3’; SEQ ID NO: 20;
Primer sg-B2M-3-down: 5’-AAACCGGAGCGAGACATCTCGGCC-3’; SEQ ID NO: 21;
Primer sg-CIITA2-up: 5’-CACCGATATTGGCATAAGCCTCCC-3’; SEQ ID NO: 22;
Primer sg-CIITA2-down: 5’-AAACGGGAGGCTTATGCCAATATC-3’; SEQ ID NO: 23;
Primer sg-TRAC-1-up: 5’-CACCAGAGTCTCTCAGCTGGTACA-3’; SEQ ID NO: 24;
Primer sg-TRAC-1-down: 5’-AAACTGTACCAGCTGAGAGACTCT-3’; SEQ ID NO: 25.

Then, using the sequences shown above as primers, anneal 2 upstream and downstream primers into 3 double stranded gRNA sequences, and add samples to the reaction system according to the instructions of Annealing Buffer for DNA Oligos (5X) (purchased from Biyuntian company).

PX330A-1X3, PX330S-2 and PX330S-3 (all purchased from Addgene Plasmid) are digested with restriction endonuclease BbsI (purchased from NEB), and the enzyme digestion reaction is carried out according to the instructions. Recover the DNA fragment after the enzyme digestion product was separated by agarose gel electrophoresis. Then, connect the corresponding annealed primer fragments and the purified vector fragments by $T_4$ ligase (purchased from Promega company) to obtain an expression vector carrying only one gRNA. After expression of *E. coli*, the plasmid is extracted with a plasmid extraction kit (from Invitrogen), and the specific method is described in the instructions.

3. According to the Golden Gate cloning technology, the above constructed expression vectors with only one gRNA are digested by restriction endonuclease BsaI(purchased from NEB company). Conduct connection reaction with quick ligase Quick Ligation™ Kit (purchased from NEB), and samples are added according to the instructions. Using X-gal and IPTG (both purchased from Biotech Biotech Co., Ltd) to screen blue and white spots, 6 combined vectors containing 3 different gRNA expression cassettes are obtained, which are shown below:

PX330A-1X3-B2M-3-CIITA2-TRAC-1, PX330-A-1X3-B2M-3-TRAC-1-CIITA2, PX330A-1X3-CIITA2-B2M-3-TRAC-1, PX330A-1X3-CIITA2-TRAC-1-B2M-3, PX330A-1X3-TRAC-1-B2M-3-CIITA2, PX330A-1X3-TRAC-1-CIITA2-B2M-3.

After expression of *E. coli,* the plasmid is extracted with a plasmid extraction kit (from Invitrogen). The vector map and PCR results are shown in Figure 2.

4. Check the correctness of the constructed vectors

The vectors obtained in step 3 are transfected into the competent cells respectively, and the sequence correctness is verified by small plasmid PCR. See the molecular cloning experiment guide (third edition, J. Sambrook et al.) for detailed steps. The experimental results are shown in Fig. 2. Three strips are visible and sequencing results are correct.

**Embodiment 3: Construction of universal CAR-T cells and phenotypic detection**

1. Determining electrotransfection conditions

Under different conditions, 293T cells are used to transfect GFP, and optimize the electrotransfection conditions. After confirming the preferred electrotransfection condition, PBMC is further transfected. The experimental results are shown in Table 2 and Table 3. The preferred electrotransfection conditions are: activation of PBMC or T cells for 48 hours, then under voltage 1000V, pulse width 35ms, conduct electric shocks twice for electrotransfection. The total number of cells is $2 \times 10^5$. After electrotransfection, fresh medium containing IL-2 is used for culture.

Table 2: Comparison of transfection efficiency and cell viability of 293T cells transfected under different electrotransfection conditions

| Serial Number | Efficiency % | Survival Rate% | Voltage/V | Pulse Width /ms | Number of Electric Shocks |
|---|---|---|---|---|---|
| 1 | 0 | 100 | 0 | 1 | 1 |
| 2 | 83.1 | 35.7 | 1400 | 20 | 1 |
| 3 | 89.8 | 27.9 | 1500 | 20 | 1 |
| 4 | 91.8 | 21.2 | 1600 | 20 | 1 |
| 5 | 85.4 | 11.8 | 1700 | 20 | 1 |
| 6 | 60.6 | 23.2 | 1100 | 30 | 1 |
| 7 | 80.3 | 27.8 | 1200 | 30 | 1 |
| 8 | 83.9 | 23.9 | 1300 | 30 | 1 |
| 9 | 90 | 14.7 | 1400 | 30 | 1 |
| 10 | 48.2 | 34.8 | 1000 | 40 | 1 |
| 11 | 73.7 | 22.4 | 1100 | 40 | 1 |
| 12 | 87.4 | 15.6 | 1200 | 40 | 1 |

(continued)

| Serial Number | Efficiency % | Survival Rate% | Voltage/V | Pulse Width /ms | Number of Electric Shocks |
|---|---|---|---|---|---|
| 13 | 65.3 | 28.6 | 1100 | 20 | 2 |
| 14 | 83.6 | 22.8 | 1200 | 20 | 2 |
| 15 | 82.6 | 20.8 | 1300 | 20 | 2 |
| 16 | 94 | 12.8 | 1400 | 20 | 2 |
| 17 | 20.3 | 42.1 | 850 | 30 | 2 |
| 18 | 47.3 | 42.8 | 950 | 30 | 2 |
| 19 | 77.3 | 32.7 | 1050 | 30 | 2 |
| 20 | 90.6 | 23.6 | 1000 | 30 | 2 |
| 21 | 78.2 | 26.3 | 1300 | 10 | 3 |
| 22 | 87.8 | 23.8 | 1400 | 10 | 3 |
| 23 | 92.3 | 14.6 | 1500 | 10 | 3 |
| 24 | 89.7 | 8.9 | 1600 | 10 | 3 |

Table 3: Electrotransfection condition optimization results of PBMC and T lymphocyte

| Cell Type | Voltage (v) | Pulse Width (ms) | Number of Electric Shocks | Number of Cells | Survival Rate /% | Efficiency /% |
|---|---|---|---|---|---|---|
| JURKAT E6-1 | 1300 | 10 | 3 | 2 x $10^5$ | -- | 25 |
| | 1500 | 20 | 1 | 2 x $10^5$ | 10.1 | 22.5 |
| | 1400 | 10 | 3 | 2 x $10^5$ | 17.6 | 23.5 |
| | 1000 | 30 | 2 | 2X$10^5$ | 18.1 | 20.3 |
| | 1000 | 35 | 2 | 2 x $10^5$ | 23.9 | 28.1 |
| PBMC-inactivated | 1000 | 30 | 2 | 2 x $10^5$ | 47.6 | 0.5 |
| PBMC-activated | 1400 | 10 | 3 | 2 x $10^5$ | 26.1 | 4.6 |
| | 1000 | 35 | 2 | 2 x $10^5$ | 28.8 | 26.5 |
| | 1000 | 30 | 2 | 2 x $10^5$ | 22.7 | 13.8 |
| | 1000 | 40 | 1 | 2 x $10^5$ | 19.6 | 7.8 |

2. Preparation of CAR-T cells from T lymphocytes infected by lentivirus containing CAR vector

1) Separation of human peripheral blood mononuclear cells

Collect about 60ml of peripheral blood using the blood collection tubes with anticoagulant, put it into 50ml centrifuge tubes, 30ml to each tube. Add 7.5ml of hydroxyethyl starch for dilution, settle naturally at room temperature (18-25 °C) for about 30min, collect the upper plasma, centrifuge for 15min, then resuspend the sediment with normal saline again, add it to the lymphocyte separation liquid according to the volume ratio of 1:1, gradient centrifuge for 20min. After centrifugation, take the second layer of white lymphocytes and wash twice with normal saline and resuspend, and the cells are cultured in RPMI 1640 complete medium containing 10% FBS to obtain human peripheral blood mononuclear cells.

2) T lymphocytes infected with lentiviral vector

The new monocyte PBMC is cultured in RPMI 1640 complete medium containing 10% FBS. After activation of anti-CD3 monoclonal antibody, the new monocyte PBMC is infected with lentivirus. Respectively add the lentiviral vectors containing CAR genes targeting CD19 or PSCA and/or the lentiviral vectors encoding HLA-E expression gene.The uninfected peripheral blood lymphocyte (PBMC) is used as the blank control. 24 hours later, collect and count cells to prepare the universal CAR-T cells, put $2 \times 10^5$ cells in each hole of the 24-hole plate, the voltage of the electrotransfection condition is 1000V, the electric pulse is 35ms, and the electrotransfection is completed by 2 electric shocks. See the operation manual of Neon ® Transfer System for the detailed steps.

3. CAR-T cell phenotype detection

Taking the targeted CD19 and PSCA as examples, the preferred electrotransfection conditions are used for electrotransfection: voltage of 1000 V, pulse width of 35 ms, 2 electric shocks. The CRISPR / cas9 expression vector of gRNA containing genes targeting B2M, CIITA and TRAC is electrotransfected into the CAR-T cells targeting CD19 or PSCA in step (2) to obtain the universal pUCAR-19 and pUCAR-PSCA cells. The T cells, which have been cultured for 10 days, infected by CAR virus, and electrotransfected to knock out related genes of TCR and MHC I and MHC II molecules by the CRISPR / cas9 system, are centrifuged for 5 minutes, at 300g/min, and the supernatant is discarded to collect the cells, and the cell density is adjusted to $1 \times 10^6$ cells /ml; the collected cells are packed separately and flow cytometry is used to detect the positive rate of protein-L to detect the positive rate of CAR expression of universal CAR-T cells. The expression of TCR and MHC I and MHC II molecules are detected by HLA-DR, B2M, CIITA, HLA-E and CD3 antibodies after PBS washing twice and removing the excess antibody uncombined with protein-L. The detection results are shown in Fig. 4. About 30% of the surface B2M and CIITA and TRAC molecules are not expressed in the modified T lymphocytes, and 43.6% of CAR is expressed as shown in Fig. 5. The method provided by the invention can simultaneously knock out 3 genes efficiently and does not affect CAR expression.

The pUCAR-CD19 or pUCAR-PSCA cells with B2M, CIITA, and TRAC molecule triple-negative and CAR positive expression are selected, Figure 6 is the obtained pUCAR-CD19 cells.

pUCAR-CD 19 cells with B2M, CIITA, and TRAC molecule triple-negative and CAR and HLA-E positive expression are selected. The triple-negative cells with CAR and HLA-E co-expression are the improved Universal CAR-T cells, represented by pUCAR-019$^{HLA\text{-}E+}$, as shown in figure 7.

4. gRNA targeting detection

Furthermore, extract the genome of the universal CAR-T cells obtained in the step 3 with the QIAamp DNA Blood Mini Kit purchased from the Qiagen (product No. 511004).

According to the instructions of the Kit, RT-PCR and sequencing are used to verify the targeted region of the designed gRNA. The results are shown in Figure 3. The bold or missing part is the gRNA targeted region designed by the R&D staff. The results show that the gRNA designed by the R&D staff can target the region of B2M, CIITA and TRAC genes to inactivate B2M, CIITA and TRAC genes, wherein - is a deletion; + is an insertion; M is a mutation.

**Embodiment 4**, **Knock out B2M, CIITA, and TRAC by CRISPR/cas9 and Off-target detection**

1. Align the gRNAs on the design website to find out the predicted off-target sites of the whole human genome. The specific genetic locus are shown in Table 4. (http://crispr.mit.edu)

2. Find the genes of the off-target sites on the NCBI website. (http://blast.ncbi.nlm.nih.gov)

3. Design the PCR primers of the corresponding genetic locus, perform PCR, connect the T vector and sequence it.

4. Analyze whether or not off-target occurs based on the sequencing results.

Table 4: Predicted off-target sites on whole human genomes

| B2M-3 | sequence | score | mismatches | | UCSC gene | locus |
|---|---|---|---|---|---|---|
| 1 | GGCCGGCATCTCTCGCTCCACGG | 0.2 | 4MMs [6:7:10:20] | AOC1 | NM_001091 | chr7:-150553540 |
| 2 | GGCCAAGATGTCTCACACCCTAG | 0.1 | 4MMs [5:15:17:20] | ZSWIM8 | NM_015037 | chr10:+75561425 |
| 3 | GGCCCAGAAGTCTGGCTCAGGAG | 0 | 4MMs [5:9:14:19] | ULK4 | NM_017886 | chr3:-41497060 |
| CIITA2 | | | | | | |
| 4 | GAAAGTTGCATCAGCCTCCCGAG | 0.5 | 4MMs [3:5:7:12] | SLC6A9 | NR_048549 | chr1:+44497045 |
| 5 | CATCTTGGCACAAGCCTCCTGGG | 0.4 | 4MMs [1:4:11:20] | RAB3D | NM_004283 | chr19:+11448071 |
| 6 | AATTTTGGCCTAAGACTCCCAAG | 0.4 | 4MMs [1:4:10:15] | QSOX1 | NM_001004128 | chr1:-180163582 |
| 7 | GATTTTGGCTGAAGCCGCCCCAG | 0.3 | 4MMs [4:10:11:17] | 27113 | NM_001127242 | chr19:-47724303 |
| TRAC-1 | | | | | | |
| 8 | TGCCTCCCTCAGCTGGTACAAGG | 0.9 | 4MMS [1:3:4:7] | HMCN1 | NM_031935 | chr1:+186039857 |
| 9 | AGTCTCTCTCAGCTGGTGCAGGG | 0.6 | 3MMs [3:4:18] | HPCAL4 | NM_016257 | chr1:+40145300 |
| 10 | AGAGTCCAGCTGCTGGTACATGG | 0.3 | 4MMs [7:8:9:11] | KIR3DL3 | NM_153443 | chr19:-55247683 |
| 11 | AGAGACTTTCATCTGGCACAGAG | 0.3 | 4MMs [5:8:12:17] | SLC26A5 | NM_198999 | chr7:-103017342 |
| 12 | AGAGTATGGCGGCTGGTACAAAG | 0.3 | 4MMs [6:8:9:11] | 3831 | NM_005552 | chr14:+104142053 |

The off-target test results are shown in Figure 8. The off-target-analysis results are 0/6 or 0/5, indicating that the gRNA designed for B2M, CIITA, and TRAC did not miss the target after repeated tests. The gRNA sequence provided by the present invention is highly specific, stable and safer in vivo.

**Embodiment 5 Verification of anti-tumor effect of universal CAR-T cells**

1. Use Hela cells with high expression of PSCA and T24 cells with no expression of PSCA as target cells to detect the validity and specificity of the universal CAR-T cells for targeting PSCA, and use pUCAR-PSCA as the effector cells planked at 1:1 ratio of effector cells to taget cells, to verify the killing effect of pUCAR-PSCA. The killing experiment is performed with the ACEA xCELLigence RTCA MP, and the procedures are carried out according to the instrument manual. On the first day, the target cells (PSCA-expressing tumor cells) are planked in a 96-well plate equipped with the instrument with 2-5*10^4 per well, and tumor cells attached to the bottom of the well are recorded every 15 minutes using the resistance index data. After 24 hours, the corresponding CAR-T cells are planked according to the pre-designed ratio of effector cells to taget cells and record resistance index data every 15 minutes, determine the proliferation or death of the adherent target cells by the resistance index. See Figure 9, the cell base is 1, and when the Y-axis is greater than 1, it shows that the tumor cell has proliferated, and when the Y-axis is less than 1, it shows that the tumor cell has been killed. T-B-C, B-T-C, T-C-B on the X-axis shows respectively the PX330A-1X3-TRAC-1-B2M-3-CIITA and PX330-A-1X3-B2M-3-TRAC-1-CIITA2 that transfected in PSCA-CAR-T cells, and also shows the universal pUCAR-PSCA cells of vector PX330A-1X3-TRAC-1-CIITA2-B2M-3. PSCA group is the autologous CAR-T cells that are infected by CAR but not transfecting the knockout system. Medium group is the normal tumor cells. PBMC group is the T lymphocytes that are neither infected by CAR nor transfecting the knockout system, the killing results are shown in Figure 9. At the $24^{th}$ hour, the universal CAR-T showed significant killing effect, especially B-T-C (px330-a-1x3-b2m-3-trac-1-ciita2) group showed high killing activity and specificity.

2. CD19-positive Raji cells (abbreviated as Raji-luc) with stably expressing firefly luciferase are used as target cells, and UCAR-19 are used as effector cells to verify the killing results of pUCAR-019 at 1:1 ratio of effector cells to taget cells; where 1 represents the pUCAR-019-1 cells indicating the CAR-019 cells infected with PX330-A-1X3-B2M-3-TRAC-1-CIITA2; 2 represents the pUCAR-019-2 cells indicating the CAR-019 cells infected with PX330A-1X3-TRAC-1-CIITA2-B2M-3. Use the standard method provided by Steady-Glo® Luciferase Assay System (Promega Cat. #E2520) kit to test the killing effect. The killing rate is calculated by the following formula.

$$\text{Cell killing rate} = (1 - \text{fluorescence intensity of co-cultivation of effector cells and target cells} / \text{fluorescence intensity of target cells}) \times 100\%$$

The killing results are shown in Fig.10, cells in pUCAR-019-1 group show high killing activity.

**Embodiment 6 Reactivity detection of universal CAR-T cells to allogeneic NK cells**

1. According to the cell size and growth speed, inoculate appropriate amount of allogeneic T-lymphocytes or pUCAR-19 cells into 96 hole plates, make sure the cell density is not more than 80-90% during test.

2. Remove the culture medium and wash once with PBS. Replace with fresh culture medium. Mix NK-92 cells with PBMC or pUCAR-19 respectively at 1:1 ratio of effector cells to taget cells and continue to culture. Take out the cell culture plate from the incubator 1 hour before the 24 hours of co-cultivation, and add LDH release reagent provided by the kit into the "sample maximum enzyme activity control hole", the amount of which is 10% of the volume of the original culture medium. After adding LDH release reagent, blow repeatedly for several times and mix well, then continue to incubate in cell incubator.

3. After 24 hours of co-cultivation, the cell culture plates are centrifuged for 5 min in a porous plate centrifuge. Take 120μl of the supernatant of each hole, and put it into the corresponding holes of a new 96 hole plate, and then test the sample.

In which, pUCAR-019 represents the triple-negative CAR cells with B2M, CIITA and TRAC knocked out and CD19 targeted. pUCAR-019$^{HLA-E+}$ represents the CAR-T cells with B2M, CIITA and TRAC knocked out to co-express HLA-E.

For the specific test method, please refer to Biyuntian Lactate Dehydrogenase Assay Kit (LDH Cytotoxicity Assay Kit), product No. C0017.

The test results are shown in Figure 11. In the LDH cytotoxicity test, after B2M is knocked out, the NK cells have significant killing effect. After knocking out B2M (HLA-I molecular universal subunit), the conserved HLA-I molecules could not be expressed. That is, they cannot protect themselves from NK cell-mediated cytotoxicity. The killing effect of the non-knockout group (PBMC) is consistent with the universal CAR-T cell group expressing HLA-E. The expression of HLA-E can compensate for the function of the conserved HLA I molecule, and inhibit the activation of NK cells. The cell lysis rates for cytotoxicity test are listed in Table 5.

Table 5: NK cell mediated cytotoxicity lysis rate of allogeneic immune cells

| Cell Types | Cell Lysis Rate 24h (%) | Cell Lysis Rate 48h (%) | Number of Repeated times |
|---|---|---|---|
| PBMC | 38.95873525 | 63.3938706 | 2 |
| pUCAR-019$^{HLA-E+}$ | 39.08128334 | 57.8858351 | 2 |
| pUCAR-019 | 51.00717304 | 81.0123622 | 2 |

Finally, the above embodiments are only used to illustrate the technical scheme of the present invention, not to limit it. Although the present invention is described in detail with reference to better embodiments, it should be understood by person skilled in the art that the technical scheme of the present invention can be modified or replaced equally, without departing from the purpose and scope of the technical scheme of the present invention, and that they should all be covered by the scope of claims in the present invention.

## Claims

1. An universal CAR-T cell, wherein both T Cell Antigen Receptor (TCR) and Major Histocompatibility Complex (MHC I, MHC II) functions of the universal CAR-T cell have been inhibited by polygenic knockout; genes encoding the TCR comprise TRAC and/or TRBC; genes encoded the Major Histocompatibility Complex comprise HLA-A, B2MH and CIITA.

2. The universal CAR-T cell according to Claim 1, wherein the TRAC, the B2M and the CIITA genes have been knocked out from said universal CAR-T cell, and the HLA-E or the HLA-F is expressed in the universal CAR-T cell.

3. The universal CAR-T cell according to Claim 1, wherein the TRAC, the HLA-A or the B2M, the CIITA genes have been knocked out from the universal CAR-T cell.

4. A method for producing the universal CAR-T cell according to anyone of Claim 1 - Claim 3, wherein the method comprises:

   1) obtain activated T cells;
   2) transfect the activated T cells of step 1) with lentiviral vector expressing CAR or co-expressing CAR and HLA-E or HLA-F, wherein the CAR is encoded to target different tumor-related molecules; and obtain CAR-T cells targeting the different tumor-related molecules;
   3) produce polygenic knockout of the CAR-T cells of step 2) by TALEN, zinc finger or CRISPR/Cas9 system methods to obtain the universal CAR-T cells, wherein knockout genes include TRAC, HLA-A and CIITA or TRAC, B2M and CIITA.

5. The method according to claim 4, where the target molecules recognized by the CAR in step 2) comprise one of CD19, PSCA, CD123, CD20, CEA (carcinoembryonic antigen), FAP, CD133, EGFR, EGFRVIII, BCMA, PSMA, Her2, CA125, EphA2, C-met, L1CAM, VEGFR, CS1, ROR1, EC, NY-ESO-1, MUC1, MUC16, mesothelin, LewisY, GPC3, GD2, EPG, DLL3, CD99, 5T4, CD22, CD30, CD33, CD138, CD171.

6. The method according to claim 4, wherein the polygenic knockout in step 3) is produced using the CRISPR/Cas9 system method.

7. The method according to claim 6, wherein the CRISPR/Cas9 system consists of gRNA and Cas9 nuclease or endonuclease.

8. The method according to claim 7, wherein electrotransfection method is preferably chosen for polygenic knockout by the CRISPR/Cas9 system, and gRNA of the knockout genes is constructed in a vector, and the knockout genes are knocked out by the electrotransfection method.

9. The method according to claim 8, wherein the gRNA sequences targeting the B2M, HLA-A, CIITA and TRAC genes are shown in SEQ ID NO: 5-19.

10. The method according to claim 9, wherein the gRNA sequences targeting the TRAC, B2M and CIITA or HLA-A, CIITA and TRAC genes are constructed on vector PX330A.

11. CRISPR/Cas9 gene knockout expression vector obtained from the method of Claim 4, wherein the expression vector comprises gene sequences shown in SEQ ID NO: 26-31.

**12.** Use of the universal CAR-T cell in the manufacture of a medicament for allogeneic treatment.

**13.** Use of the universal CAR-T cells in the manufacture of a medicament for malignant tumor or infectious disease.

**14.** The use of Claim 13, wherein cells or tissues of the malignant tumor or the infectious disease are capable of expressing one of CD19, PSCA, CD123, CD20, CEA (carcinoembryonic antigen), FAP, CD133, EGFR, EGFRVIII, BCMA, PSMA, Her2, CA125, EphA2, C-met, L1CAM, VEGFR, CS1, ROR1, EC, NY-ESO-1, MUC1, MUC16, mesothelin, LewisY, GPC3, GD2, EPG, DLL3, CD99, 5T4, CD22, CD30, CD33, CD138, CD171.

**15.** The use of Claim 13, wherein the malignant tumor or the infectious disease comprise: hematological tumor, solid tumor, immune rejection caused by allogeneic transplantation, and autoimmune diseases such as allergic reaction or systemic lupus erythematosus.

**16.** Universal T cell used in the manufacture of a medicament for allogeneic treatment, wherein functions of T cell Antigen Receptor (TCR) and Major Histocompatibility Complex (MHC I, MHC II) are simultaneously inhibited in the universal T cell.

**17.** The universal T cell according to Claim 16, wherein genes encoding the TCR include TRAC and/or TRBC, and genes encoding the Major Histocompatibility Complex include HLA, B2MH and CIITA.

**18.** The universal T cell according to Claim 16, wherein the universal T cell expresses HLA-E or HLA-F; and TRAC, HLA-A and CIITA genes or TRAC, B2M and CIITA genes have been knocked out.

**19.** Use of the universal T cell of anyone of Claim 16 - Claim 18 in the manufacture of a medicament for malignant tumor or infectious disease.

**20.** The use of Claim 19, wherein the malignant tumor or the infectious disease comprises: hematological tumor, solid tumor, immune rejection caused by allogeneic transplantation, and autoimmune diseases such as allergic reaction or systemic lupus erythematosus.

| Sample ID | Client ID | HLA-A* | HLA-B* | HLA-C* |
|---|---|---|---|---|
| R-XNYY160449 | HD-01 | A*01:01:01:01 A*02:01:01:01 | B*37:01:01 B*52:01:01 | C*06:02:01 C*12:02:02 |
| R-XNYY160450 | HD-002 | A*02:01:01:01 A*24:02 | B*40:01 B*54:01:01 | C*01:02:01 C*07:02:01 |
| R-XNYY160451 | HD-03 | A*02:01:01:01 A*02:05:01 | B*13:02:01 B*50:01:01 | C*06:02:01 C*06:02:01 |
| R-XNYY160452 | HD-04 | A*03:01:01:01 A*30:01:01 | B*13:02(NEW) B*35:01(NEW) | C*04:01:01G C*06:02:01 |
| R-XNYY160453 | HD-05 | A*02:01:01:01 A*11:01:01:01 | B*39:01 B*54:01:01 | C*01:02:01 C*07:02:01 |
| R-XNYY160454 | HD-06 | A*11:01:01:01 A*33:03:01 | B*40:01 B*58:01:01:01 | C*03:02:01 C*07:02:01 |
| R-XNYY160455 | HD-07 | A*02:01:01:01/A*02:06:01:01 A*02:10/A*02:242 | B*40:06:01:01 B*46:01:01 | C*01:02:01 C*08:01:01G |
| R-XNYY160456 | HD-08 | A*02:07:01 A*11:01:01:01 | B*15:02:01 B*15:02:01 | C*08:01:01G C*08:01:01G |
| R-XNYY160457 | HD-09 | A*02:07:01 A*11:01:01:01 | B*15:01:01:01 B*40:06:01:01 | C*03:04:01 C*04:01:01:01 |
| R-XNYY160458 | HD-11 | A*02:01:01:01 A*24:02 | B*15:01:01:01 B*46:01:01 | C*01:02:01 C*04:01:01:01 |
| R-XNYY160459 | HD-12 | A*11:01:01:01 A*30:01:01 | B*13:02:01 B*38:02:01 | C*06:02:01 C*07:02:01 |
| R-XNYY160460 | HD-14 | A*02:03:01 A*11:01:01:01 | B*15:02:01 B*40:01 | C*07:02:01 C*08:01:01G |
| R-XNYY160461 | HD-15 | A*11:01:01:01 A*24:02 | B*39:01 B*55:02:01:01 | C*01:02:01 C*07:02:01 |
| Results: R-XNYY160452, there is a mutation of C → A at 2946bp position of exon2 in B*13:02 or B*35:01 of HD-04. | | | | |

Figure 1

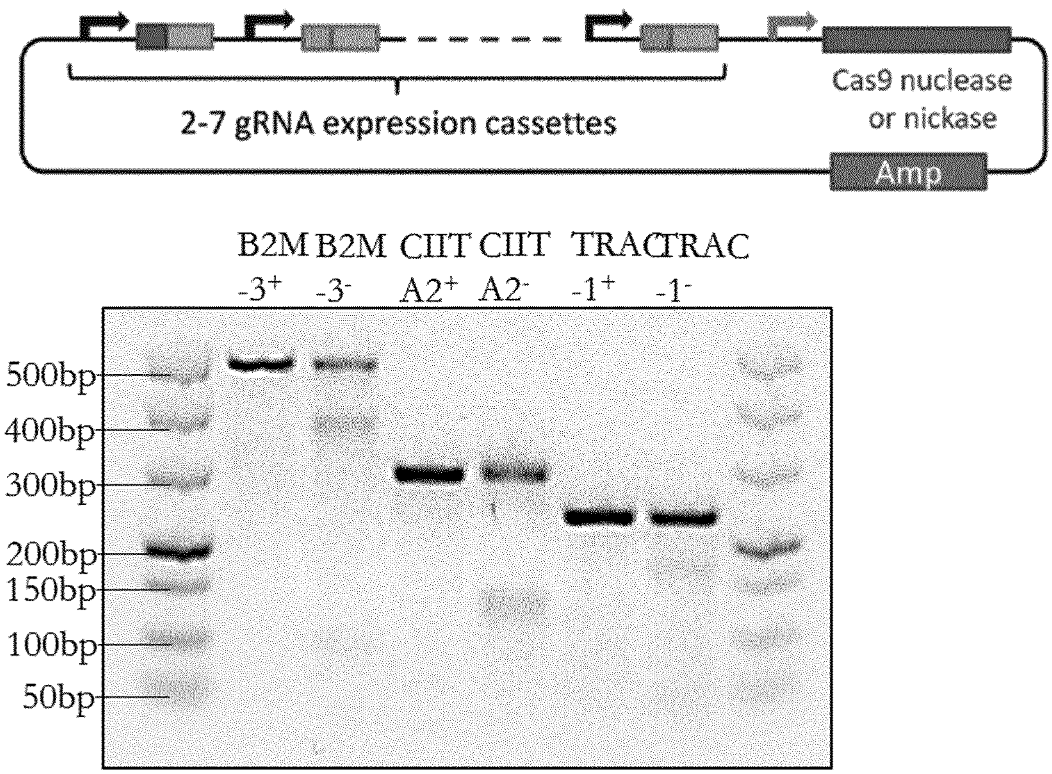


Figure 2

| | | |
|---|---|---|
| B2M-3 | TGACAGCATTCGGGCCGAGATGTCTCGCTCCGTGGCCTTAGCTGTGCT | download |
| WT | TGACAGCATTCGGGCCGAGATGTCTCGCTCCGTGGCCTTAGCTGTGCT | |
| 16. 17. 19. 1. 2. 3. 5. 8. 9. 10. 13 | TGACAGCATTCGGGCCGAGATGTCTCGCTTCCGTGGCCTTAGCTGTGCT | +1 |
| 20 | TGACAGCATTCGGGCCGAGATGTCTCG--TCCGTGGCCTTAGCTGTGCT | -1 |
| 4 | TGACAGCATTCGGGCCCGCCGTGGGGCTAGTCCAGGGCTTAGGCCTTAGCTGTGCT | M9+8 |
| 7 | -------------------------------------------CCTTAGCTGTGCT | -46 |
| 11 | TGACAGCATTCGGGCCGAGATGTCTCGC-CCGTGGCCTTAGCTGTGCT | -1 |
| 12 | TGACAGCATTCGGGCCGAGATGT----------GGCCTTAGCTGTGCT | -10 |
| CIITA2 | GGAAGGTGATGAAGAGACCAGGGAGGCTTATGCCAATATCGGTGAGGAAG | download |
| WT | GGAAGGTGATGAAGAGACCAGGGAGGCTTATGCCAATATCGGTGAGGAAG | |
| 7 | GGAAGGTGATGAAGAGACCAGGGA---TTATGCCAATATCGGTGAGGAAG | -3 |
| 8 | GGAAGGTGATGAAGAGACCAGGGCATAAGGCTTATGCCAATATCGGTGAGGAAG | +5 |
| 14 | GGAAGGTGATGAAGAGACCAGG---------CCAATATCGGTGAGGAAG | -9 |
| 28 | GGAAGGTGCTT-----------CC---------T------------------------ | -36 |
| 34 | GGAAG--------------------------------------------- | -45 |
| 1 | GGAAGGTGATGAAGAGACCAG-----------------------AGGAAG | -23 |
| TRAC | CCTGACCCTGCCGTGTACCAGCTGAGAGACTCTAAATCCAGTG | download |
| WT | CCTGACCCTGCCGTGTACCAGCTGAGAGACTCTAAATCCAGTG | |
| 21. 15 | CCTGACCCTGCCGTGTTACCAGCTGAGAGACTCTAAATCCAGTG | +1 |
| 22. 23. 24. 26. 27. 28. 6. 7. 9. 10. 13. 14. 18 | CCTGACCCTGCCGTGTAACCAGCTGAGAGACTCTAAATCCAGTG | +1 |
| 25 | CCTGACCCTGCCGTG--ACAAG----------TCTAAATCCAGTG | -10/M1 |
| 1 | CCTGACCCTG------ACCAGCTGAGAGACTCTAAATCCAGTG | -6 |
| 2 | CCTGACCCTGCCC-------GCTGAGAGACTCTAAATCCAGTG | M1-7 |
| 11 | CCTGACCCTGC--------AGCTGAGAGACTCTAAATCCAGTG | -8 |
| 17 | CCTGACCCTGCCGTGTA------GAGAGACTCTAAATCCAGTG | -6 |

Figure 3

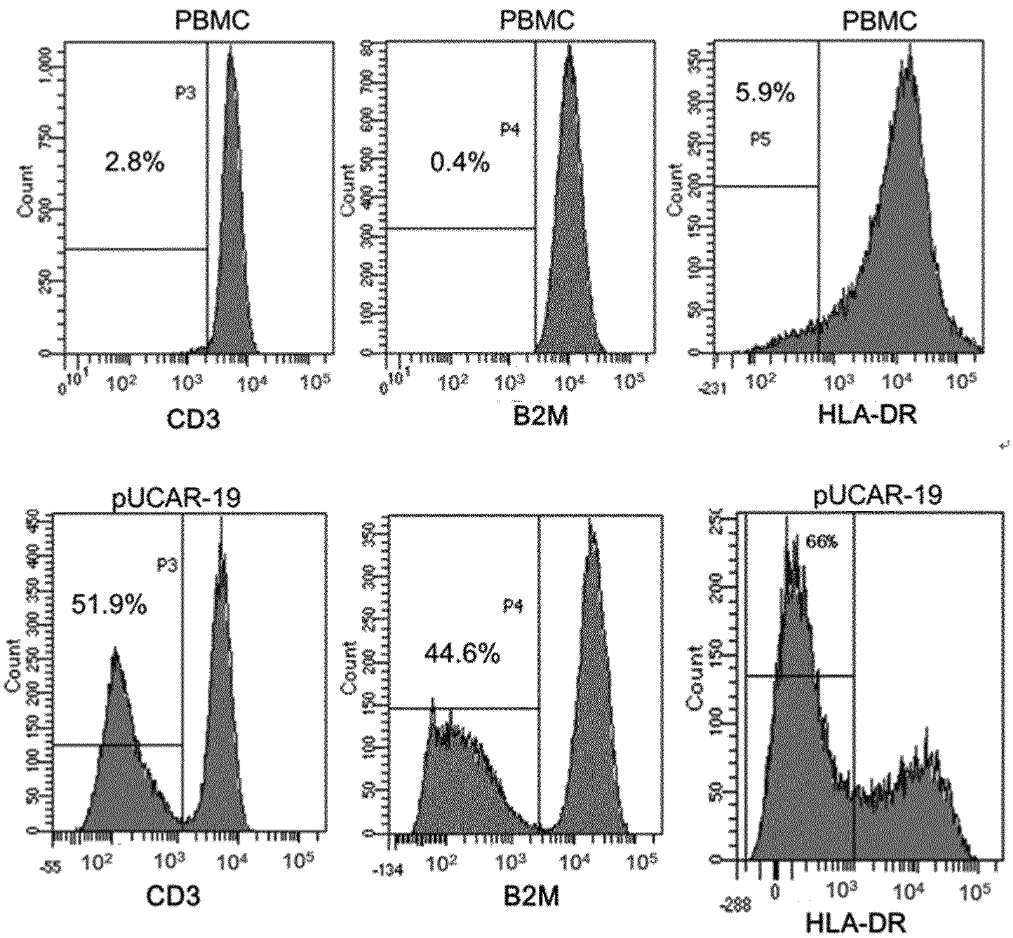


Figure 4

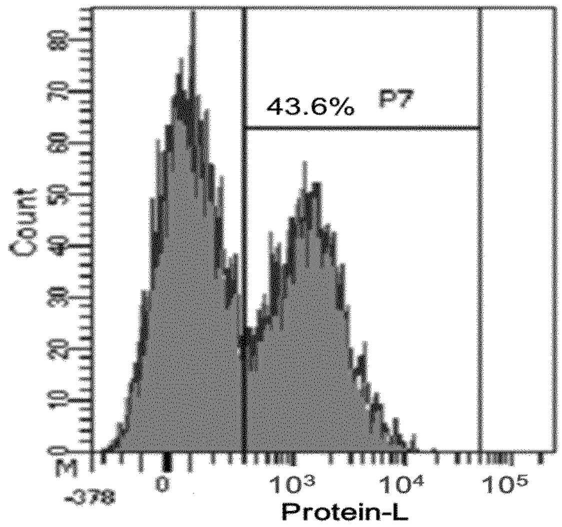


Figure 5

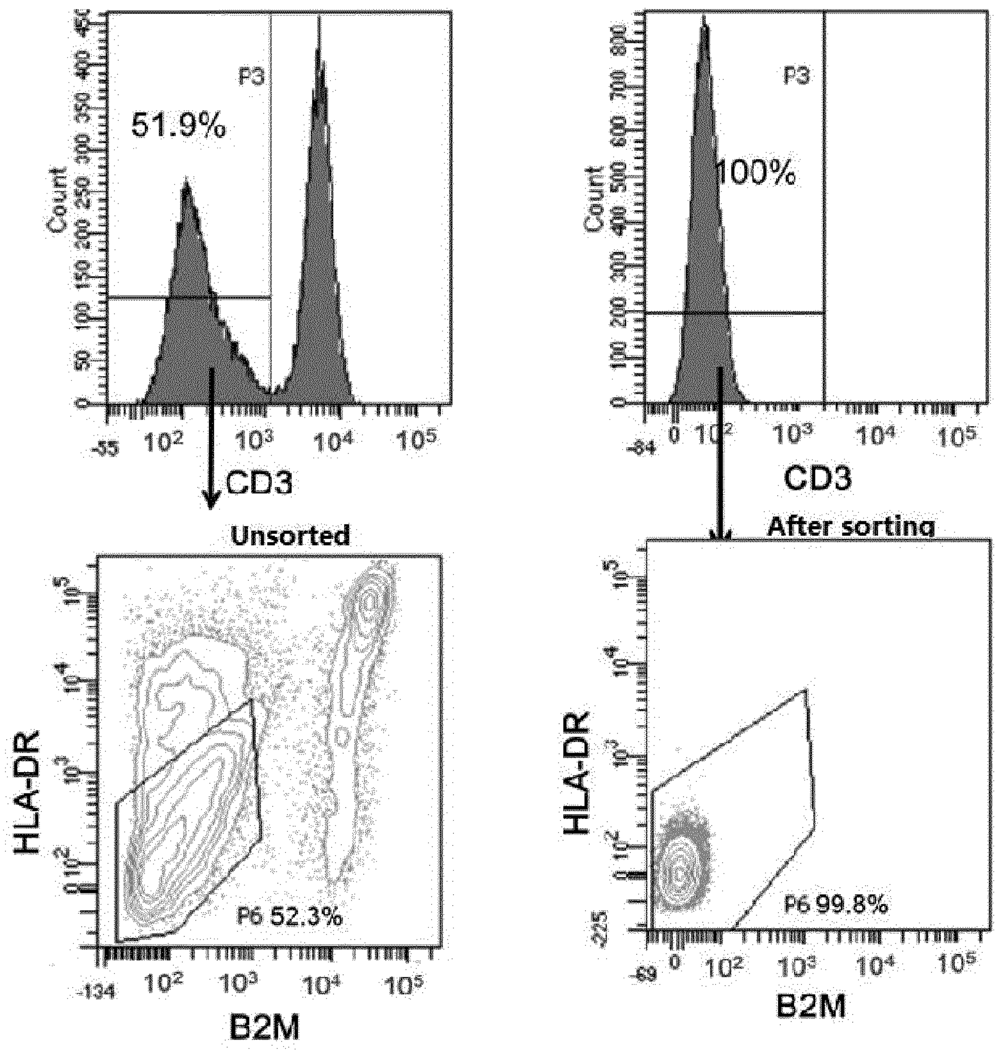


Figure 6

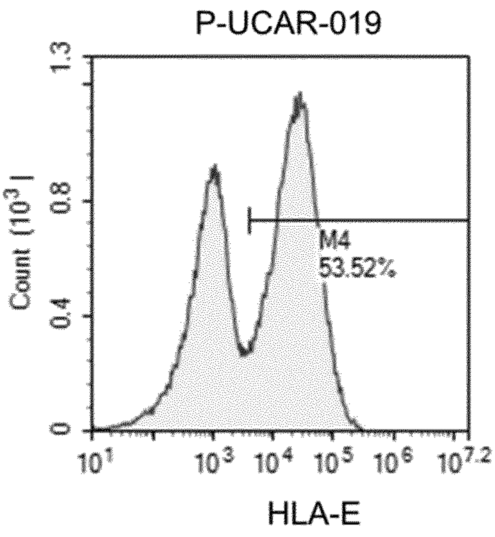


Figure 7

| Target site | gRNA motif (mismatches in red) | PAM | Gene | Off-target analysis |
|---|---|---|---|---|
| sgB2M-3 | GGCCGAGATGTCTCGCTCCG | TGG | B2M | |
| Off-target1 | GGCCGGCATCTCTCGCTCCA | CGG | AOC1 | 0/6 |
| Off-target2 | GGCCAAGATGTCTCACACCC | TAG | ZSWIM8 | 0/6 |
| Off-target3 | GGCCCAGAAGTCTGGCTCAG | GAG | ULK4 | 0/5 |
| **Target site** | **gRNA motif (mismatches in red)** | **PAM** | **Gene** | **Off-target analysis** |
| sgCIITA2 | GATATTGGCATAAGCCTCCC | TGG | CIITA | |
| Off-target5 | CATCTTGGCACAAGCCTCCT | GGG | RAB3D | 0/6 |
| Off-target6 | AATTTTGGCCTAAGACTCCC | AAG | QSOX1 | 0/5 |
| Off-target7 | GATTTTGGCTGAAGCCGCCC | CAG | 27113 | 0/5 |
| **Target site** | **gRNA motif (mismatches in red)** | **PAM** | **Gene** | **Off-target analysis** |
| sgTRAC-1 | AGAGTCTCTCAGCTGGTACA | CGG | TRAC | |
| Off-target8 | TGCCTCCCTCAGCTGGTACA | AGG | HMCN1 | 0/6 |
| Off-target9 | AGTCTCTCTCAGCTGGTGCA | GGG | HPCAL4 | 0/6 |
| Off-target10 | AGAGTCCAGCTGCTGGTACA | TGG | KIR3DL3 | 0/6 |
| Off-target11 | AGAGACTTTCATCTGGCACA | AGG | SLC26A5 | 0/6 |
| Off-target12 | AGAGTATGGCGGCTGGTACA | AAG | 3831 | 0/6 |

Figure 8

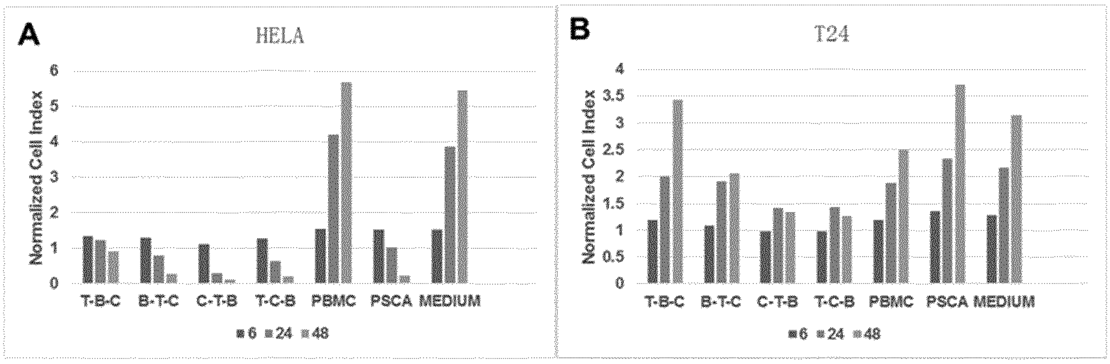


Figure 9

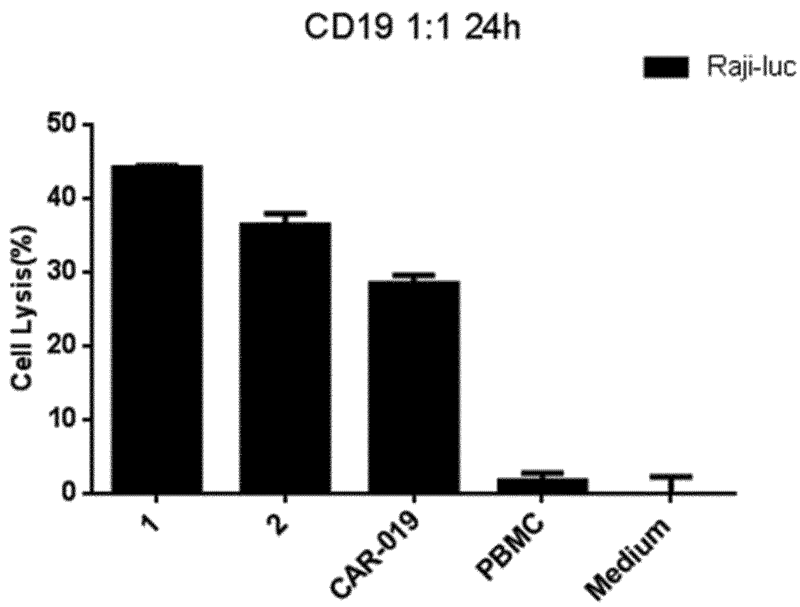


Figure 10

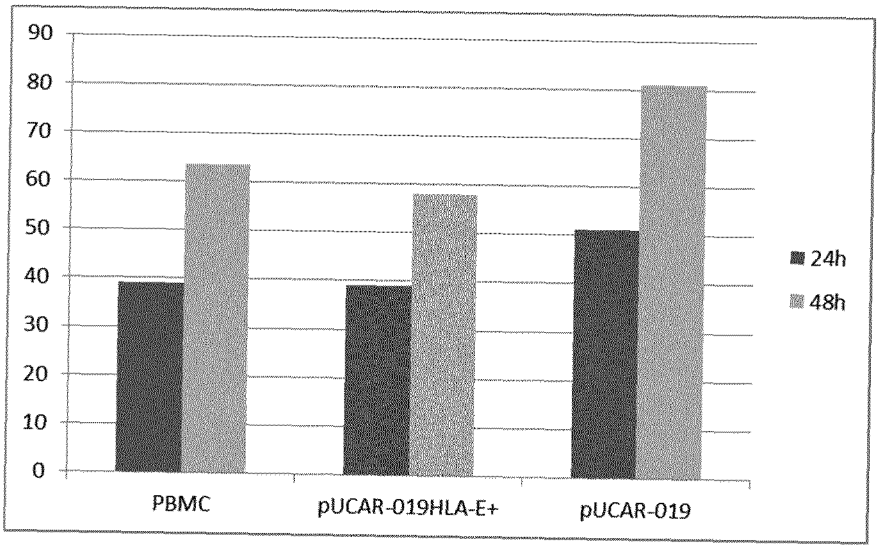


Figure 11

**INTERNATIONAL SEARCH REPORT**

International application No.
**PCT/CN2018/103714**

**A. CLASSIFICATION OF SUBJECT MATTER**

C12N 5/10(2006.01)i; C12N 15/867(2006.01)i; A61K 35/17(2015.01)i; A61P 35/00(2006.01)i; A61P 31/00(2006.01)i; A61P 37/06(2006.01)i; A61P 37/08(2006.01)i; A61P 37/02(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS, VEN (DWPI+SIPOABS), CNTXT, EPTXT, USTXT, WOTXT, CNKI, 百度学术, Baidu, ISI-WEB OF SCIENCE, Genbank+EMBL, 中国专利生物序列检索系统, China Patents Biological Sequence Search System: T细胞, 抑制, T细胞抗原受体, 主要组织相容?复合?, 敲除, T cell?, T lymphocyte, inhibit+, T cell antigen receptor, TCR, main histocompatibility complex, MHC, TRAC, TRBC, HLA-A, B2M, β2M, CIITA, HLA-E, HLA-F, 对序列SEQ ID NO:5-19和26-31的检索, search for SEQ ID NOs: 5-19 and 26-31

**C. DOCUMENTS CONSIDERED TO BE RELEVANT**

| Category* | Citation of document, with indication, where appropriate, of the relevant passages | Relevant to claim No. |
|---|---|---|
| PX | CN 107723275 A (CHONGQING PRECISION BIOTECHNOLOGY CO., LTD.) 23 February 2018 (2018-02-23)<br>see claims 1-20, and description, paragraphs [0007]-[0057] | 1-20 |
| X | WO 2017093969 A1 (NOVARTIS AG ET AL.) 08 June 2017 (2017-06-08)<br>see abstract, and description, p. 1, line 23 to p. 65, line 10 and p. 690, line 34 to p. 691, line 5 | 1-20 |
| A | XU, Jing et al. "T细胞介导的精准肿瘤免疫治疗 (T cell-mediated precision cancer immunotherapy)"<br>*中国肿瘤生物治疗杂志 (Chinese Journal of Cancer Biotherapy),*<br>Vol. 23, No. 5, 20 October 2016 (2016-10-20),<br>ISSN: 1007-385X,<br>see pp. 714-719 | 1-20 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 November 2018** | **30 November 2018** |
| Name and mailing address of the ISA/CN<br>**State Intellectual Property Office of the P. R. China (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

INTERNATIONAL SEARCH REPORT

International application No.
PCT/CN2018/103714

C. DOCUMENTS CONSIDERED TO BE RELEVANT

| Category* | Citation of document, with indication, where appropriate, of the relevant passages | Relevant to claim No. |
|---|---|---|
| A | Poirot, L. et al. "Multiplex Genome-Edited T-cell Manufacturing Platform for "Off-the-Shelf" Adoptive T-cell Immunotherapies" *CANCER RESEARCH,* Vol. 75, No. 18, 15 September 2015 (2015-09-15), ISSN: 0008-5472, see pp. 3853-3864 | 1-20 |

Form PCT/ISA/210 (second sheet) (January 2015)

INTERNATIONAL SEARCH REPORT

International application No.
**PCT/CN2018/103714**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**
**Information on patent family members**

International application No.
**PCT/CN2018/103714**

| Patent document cited in search report | | | Publication date (day/month/year) | Patent family member(s) | | | Publication date (day/month/year) |
|---|---|---|---|---|---|---|---|
| CN | 107723275 | A | 23 February 2018 | None | | | |
| WO | 2017093969 | A1 | 08 June 2017 | IL | 259576 | D0 | 31 July 2018 |
| | | | | CA | 3006432 | A1 | 08 June 2017 |
| | | | | AU | 2016362129 | A1 | 14 June 2018 |
| | | | | SG | 11201804373V | A | 28 June 2018 |
| | | | | EP | 3384027 | A1 | 10 October 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **J. SAMBROKE.** Molecular Cloning Experiment Guide
- **J. SAMBROOK.** the molecular cloning experiment guide